# EUROPEAN PATENT APPLICATION

(11) **EP 4 541 782 A1**
(43) Date of publication of application: **23.04.2025**
(21) Application number: 24203107.8
(22) Date of filing: 27.09.2024
(51) Int. Cl.: C07C 37/20, C07C 39/16, C07C 37/84

(54) **PRODUCTION PROCESS FOR SYNTHESIZING BISPHENOL A BY A RESIN METHOD**

(30) Priority: 16.10.2023 CN 202311333944
(71) Applicant: Tianjin University, Tianjin 300072 (CN)
(72) Inventor: ZHANG, Minhua, Tianjin, 300072 (CN); GONG, Hao, Tianjin, 300072 (CN); DONG, He, Tianjin, 300072 (CN); SHI, Feng, Tianjin, 300072 (CN); GENG, Zhongfeng, Tianjin, 300072 (CN); YU, Yingzhe, Tianjin, 300072 (CN); JIANG, Haoxi, Tianjin, 300072 (CN)
(74) Representative: Cabinet Beau de Loménie

(57) **Abstract**

The present invention relates to a production process and device for synthesizing bisphenol A by a resin method, including a condensation reaction unit for performing a catalytic condensation reaction between phenol and acetone to generate bisphenol A, a first-stage adduct crystallization unit for mixing a concentrated solution with adduct crystals recycled after melting and then performing adduct crystallization, a liquid-phase dephenolization unit for melting an adduct to yield a bisphenol A product, a secondary adduct crystallization unit for recycling phenol and bisphenol A from a mother solution, a solvent recycling unit for recycling unreacted phenol and unreacted acetone, and discharging phenol-containing process water for sewage treatment, and a cracking and rearrangement unit for recycling the phenol from the mother solution, and convert bisphenol A, 2,4-bisphenol A and the like therein in the mother solution into the bisphenol A.

## Description

### TECHNICAL FIELD

The present invention relates to a process for producing bisphenol A, in particular to a production process and device for synthesizing bisphenol A by a resin method.

### BACKGROUND ART

Bisphenol-A (Bisphenol-A, BPA) has a molecular formula of C₁₅H₁₆O₂ and a molecular weight of 228.29, and has a chemical structural formula:

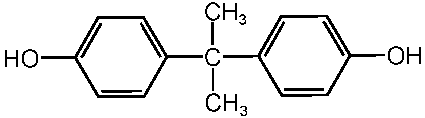

The bisphenol A is a white crystal, which is dissolved in ethanol, diethyl ether and acetic anhydride, has low solubility in cold water (weight ratio of 1: 2820), and is slightly soluble in boiling water (weight ratio of 1: 130). The bisphenol A is capable of forming crystalline adducts with various inorganic and organic substances (e.g., phenols, isopropanol, amines, and ammonia), with components typically present in equimolar ratios. These adducts are highly unstable and may cause decomposition, whether by washing with water or by heating under normal pressure, vacuum or in the presence of inert gases.

The bisphenol A, serving as one of important organic chemical raw materials, is a main raw material for producing epoxy resin and polycarbonate, and can also be used for preparing high polymer materials such as polysulfone resin, phenolic unsaturated resin, polyarylate compounds, polyetherimide, polyvinyl chloride heat stabilizer and rubber antioxidant.

Currently, the production of the bisphenol A primarily involves a condensation reaction between acetone and phenol in the presence of an acidic catalyst, the phenol being in large excess; and the representative acidic catalyst used is strongly acidic cation exchange resin. A mixture produced from the reaction contains, besides the bisphenol A, more than ten substances such as unreacted phenol, acetone, water produced from the reaction, isomers of the bisphenol A, triphenols, and chromans. It is well-known that the bisphenol A producing process is as follows: crystallizing the mixture resulting from the condensation reaction, followed by solid-liquid separation, washing crude crystals, and removing the phenol from the crude crystals to allow for recycling the phenol.

CN200910030415.2 has disclosed a method for circularly enhancing a bisphenol A concentration and reducing energy consumption by utilizing a reaction liquid. According to the method, after the acetone, the water and part of phenol are removed from the reaction liquid, the reaction liquid is fed into a vacuum evaporation system to remove the phenol, a high-purity bisphenol A molten solution is yielded through a circulation process of crystallization and a circulation process of melting for several times, and materials are allowed to return to a reactive synthesis inlet after vacuum dehydration.

CN202111654111.0 has disclosed an optimization method for preparing a bisphenol A reaction system. The optimization method includes the following steps of allowing a mother solution yielded by treating at least one part of post-reaction liquid to undergo the following reaction processes in sequence: isomerization, concentration, partial pre-concentration, cracking and rearrangement; and after the bisphenol A preparation reaction system operates, simultaneously performing the isomerization reaction and the rearrangement reaction in one isomerization-rearrangement reactor to form a cyclic operation system, and allowing isopropyl phenol generated from the cracking process to enter a phenol recycling system along with light components and be removed from the phenol along with mixed polyphenol in the partial pre-concentration process.

CN202111165633.9 has disclosed a method for reducing isopropyl phenol in a bisphenol A preparation reaction system, including the following steps: performing a condensation reaction between excessive phenol and acetone in a condensation reactor through a catalyst, performing isomerization-cracking-rearrangement on at least one part of post-reaction liquid to yield a rearrangement solution, and performing concentration process treatment on the rearrangement solution containing the isopropyl phenol, allowing the isopropyl phenol to enter a phenol recycling system to be separated from the phenol along with mixed polyphenol; wherein the mass percent content of the isopropyl phenol in the condensation reactor is less than or equal to 0.25%.

In summary and based on other references, during the production process of the bisphenol A, most of reaction systems involve one-stage or two-stage tubular reactors that are connected in series, reaction heat is released to continuously elevate a temperature of the system along with the progress of the reaction, and the content of byproduct water will also continuously increase. In the presence of high temperature and water, the progress of a forward reaction will be inhibited, and the equilibrium conversion rate will be further reduced, preventing the reaction from reaching the ideal rate and conversion rate.

From the perspective of the reaction system, currently, a method for increasing the conversion rate of the phenol from a return stream of a separation section into the reactor is involved, but no effective method for solving excessively high temperature in the reactor has been proposed; and a rectification tower is used for removing light components behind the reactor, so that water participates in the full course of the reaction, which is unfavorable for the forward reaction, and the rectification tower has higher energy consumption and is not suitable for separating substances herein;

from the perspective of a separation system, the current process involves multiple steps of crystallization, washing, and melting, which is tedious and repetitive. The fundamental cause is that the efficiency of solid-liquid separation equipment is low, and the separation objective of the system cannot be met;

from the perspective of overall energy utilization, the whole process has more endothermic processes and exothermic processes, including preheating, condensation, evaporation and the like, wherein the energy utilization efficiency varies significantly among different evaporation methods; however, in typically simple first-stage evaporation or first-stage climbing/falling film evaporation used in a plurality of industrial examples and patents, energy is not utilized fully in a gradient manner, and heat exchange between streams is less, leading to serious energy waste; and

from the perspective of overall material circulation, the circulating streams between sections are unreasonable in configuration, and has the defect of more serious material backmixing, leading to an increase in the burdens on a separating unit and power equipment.

In summary, the existing production process for the bisphenol A has various aspects of defects, ranging from a local aspect to an overall process, and in order to improve the purity of products and reduce energy consumption, it is necessary to adjust the trend of part of circulating streams throughout the process, increase heat exchange between process streams throughout the process, and focus on improving part of processes and devices. The present invention is as follows.

### SUMMARY OF THE INVENTION

The present invention provides a process and device for synthesizing bisphenol A. By arranging an external circulation heat exchanger of a reactor, a light component removing tank between two stages of reactions, a pressurized separating unit, a double-effect falling film evaporator and an isomerization reactor, while adjusting the trend of part of recycling streams in the overall process, and increasing heat exchange between process streams in the overall process, the objectives of improving the purity of products and circulating materials, reducing energy consumption and improving the selectivity of the bisphenol A are fulfilled.

A production process for synthesizing bisphenol A by a resin method includes the following aspects:
(1) the production process includes a condensation reaction unit configured to perform a catalytic condensation reaction between phenol and acetone to generate bisphenol A in the presence of a resin catalyst, and a reactor of the condensation reaction unit includes first-stage reactors and second-stage reactors, wherein external circulation heat exchange is arranged in the first-stage reactors, and a flash evaporation dehydration process is arranged between the first-stage reactors and the second-stage reactors;
(2) the production process includes a first-stage adduct crystallization unit and a process of separating a solid phase from a liquid phase with a pressurized separating unit, wherein the adduct crystallization unit is configured to mix a concentrated solution from the condensation reaction with adduct crystals recycled after melting and then perform adduct crystallization, filter by a rotary drum under pressure, add phenol in the solution to mix well, and wash the adduct crystals, and perform centrifugal separation to yield adduct crystals;
(3) the production process includes a liquid-phase dephenolization unit configured to perform phenol removal, granulation and packaging on an adduct molten solution to yield a bisphenol A product;
(4) the production process includes a second-stage adduct crystallization unit and a process of separating the phenol from the bisphenol A through a double-effect falling film evaporator and converting a byproduct into the bisphenol A through an isomerization reactor, wherein the second-stage adduct crystallization unit is configured to recycle the phenol and the bisphenol A from a mother solution;
(5) the production process includes a solvent recycling unit configured to recycle unreacted phenol and unreacted acetone, and discharge phenol-containing process water for sewage treatment; and
(6) the production process includes a cracking and rearrangement unit configured to remove impurities generated in the production process, recycle the phenol from the mother solution, and convert the bisphenol A and 2,4-bisphenol A in the mother solution into the bisphenol A that returns to the production process through cracking and rearrangement.

The production flow of synthesizing bisphenol by the resin method is as follows: feeding raw material acetone, recycled acetone and circulating phenol into the condensation reaction unit (100#) for a reaction, simple separating a product from an unreacted raw material mixture, feeding a reaction liquid into the first-stage adduct crystallization unit (200#), and feeding aqueous phenol into the solvent recycling unit (500#); introducing part of raw material phenol from the first-stage adduct crystallization unit for washing an upstream reaction liquid, recycled phenol from a dephenolization refining unit (300#) and the adduct crystal molten solution from a mother solution recycling unit (400#), after washing, feeding a first-stage centrifugal mother solution/washing solution and a second-stage centrifugal mother solution into the mother solution recycling unit, and feeding the adduct crystal molten solution into the dephenolization refining unit; feeding the aqueous phenol into the solvent recycling unit while yielding a final product BPA through the dephenolization refining unit; introducing part of raw material phenol from the mother solution recycling unit for washing the mother solution, the washing solution, the recycled phenol and the recycled BPA from the cracking and rearrangement unit (600#), followed by feeding the second-stage centrifugal mother solution into the cracking and rearrangement unit; adding ethylbenzene to the solvent recycling unit to serve as an entrainer, and discharging effluent out of the system while feeding the recycled acetone into the condensation reaction unit and feeding the recycled phenol into the mother solution recycling unit; and receiving the second-stage centrifugal mother solution by the cracking and rearrangement unit to perform cracking and rearrangement, feeding the recycled BPA back to the mother solution recycling unit, and delivering p-isopropyl phenol and effluent out of the system.

According to the condensation reaction unit (100#), refined fresh acetone, at flow of 14000-16000 kg/h, is respectively fed into the first-stage reactor (102) and the second-stage reactor (112) by weight in proportion; the acetone fed into the first-stage reactor (102) is mixed with the recycled phenol from a cracking feed preheater (601) and a material from a first-stage circulating pump (105), a mixture enters a first-stage cooler (101), and enters the first-stage reactors (102-104) after being cooled down by circulating water; one part of the reaction liquid flowing out of the first-stage reactor (104) circulates back to the first-stage reactor (102) through the first-stage circulating pump (105), with circulating flow of 455000-520000 kg/h, and the remaining reaction liquid is heated by a dehydration flash preheater (106) and enters a dehydration flash tank (107) to remove part of water from the remaining reaction liquid; flash gas from the dehydration flash tank (107) is condensed and cooled down by a first flash condenser (108) and a second flash condenser (109), and a condensate containing water, acetone and phenol automatically flows to a light component receiving tank (124), while tail gas is fed into a vacuum system; a material dehydrated from the dehydration flash tank (107) is mixed with the recycled acetone and a second stream of fresh acetone, the mixed material is then fed into a second-stage cooler (111) through a second-stage feed pump (110), and enters second-stage reactors (112-114) after being cooled down by the circulating water; a condensation reaction liquid from the second-stage reactor (114) is mixed with tower bottoms from a phenol absorption tower (306), then a mixture enters an adsorption column (115), and passes through a de-light component tower feed preheater (116) to be heated by heat exchange with tower bottoms of a de-light component tower (117) before entering the de-light component tower (117); the water, the unreacted acetone and part of the phenol in the reaction liquid are distilled out of a tower overhead, and after condensation through a de-light component tower condenser (122) and a de-light component tower aftercooler (123), tail gas enters the vacuum system, a condensate automatically flows into the light component receiving tank (124) and is delivered to the solvent recycling unit 500# through a discharge pump (125) for recycling the phenol and the acetone; and a de-light component tower reboiler (118) is configured to concentrate a material in a tower kettle through heating by low-pressure vapor, and the concentrated material is pumped out by a tower kettle discharge pump (119) of the de-light component tower, cooled down by heat exchange in the de-light component tower feed preheater (116), and delivered to a condensation reaction liquid receiving tank (120).

According to the first-stage adduct crystallization unit (200#), a reaction concentrated solution from a condensation reaction liquid discharge pump (121) and a recycled adduct crystal molten solution of the mother solution recycling unit (400#) from a melt crystallizer circulating pump (427) are mixed in a crystallization feed buffer tank (201), then pumped out by a crystallizer feed pump (202) and cooled down by a primary crystallizer feed cooler (203) and enters a primary crystallization circulator (204); slurry is pumped out of the primary crystallization circulator (204) by a crystallization circulating pump (205), and then enters a crystallization cooler (206), and heat of crystallization is removed from the slurry under a cooling effect of deionized water; the slurry flowing out of the primary crystallization circulator (204) is continuously delivered to a first-stage primary pressurized separating unit (207) for solid-liquid separation through a flow control valve; the phenol for washing a filter cake is a second-stage centrifuge filtrate from a slurry mixing washing mother solution pump (214), and the mother solution separated from the slurry and the washing solution enter a mother solution/washing solution tank (208) of the primary separating unit and pumped to a mother solution receiving tank (401) through a dephenolization feed pump (218); the phenol from a phenol discharge pump (311) is introduced from the primary separating unit, and the phenol and the filtered adduct crystals undergo mixing and washing in a slurry mixing washing tank (210); the slurry undergoing slurry mixing is respectively pumped to a second-stage primary pressurized separating unit (212) for solid-liquid separation through a corresponding slurry pump (211); a filtrate enters a slurry mixing centrifuge mother solution tank (213), is pumped out by the slurry mixing washing mother solution pump (214) and is respectively delivered to the first-stage primary pressurized separating unit (207) and a secondary pressurized separating unit (423) for washing the phenol; the filter cake is removed by a centrifuge and fed into a melt crystallizer (215); and a material in the melt crystallizer (215) is pumped to a melt heater (217) by a corresponding melt crystallizer circulating pump (216) to be heated, and then returns to the melt crystallizer (215).

According to the dephenolization refining unit (300#), an adduct molten solution from a dephenolization feed pump (218) enters a falling film dephenolizer (301), is vaporized under the heating of water vapor, and enters a gas-liquid separation tank (302); a liquid phase enters a tower overhead of a stripper dephenolization tower (305); a gaseous phase is condensed by a stripper dephenolization tower condenser (303), tail gas enters the vacuum system, and a condensate enters a phenol tank (310); fresh phenol from a tank zone enters a fresh phenol tank (313) at flow of 42756-48864 kg/h, and is pumped out by a fresh phenol pump (314), one part of the fresh phenol is fed into the phenol tank (310) after being metered, and the remaining fresh phenol is delivered to a phenol recycling tank (416); the phenol in the phenol tank (310) is pumped out by a phenol discharge pump (311), cooled down to a set value by a phenol washing cooler (312), and is then delivered to the slurry mixing washing tank (210) used for washing; the water vapor enters a tower bottom of the stripper dephenolization tower (305) as stripped vapor after being heated by an electrically heated vapor superheater (304); liquid bisphenol A yielded at the tower bottom is pumped out by a BPA melt pump (315) and then formed and granulated, and the granulated bisphenol A enters a packaging machine (316) for packaging, so as to yield a bisphenol A product; a gaseous phase at a tower overhead enters a tower bottom of a phenol absorption tower (306) and performs gas-liquid mass transfer with the absorbed phenol added from a tower overhead; the gas discharged from the tower overhead enters a phenol absorption tower condenser (309), tail gas enters the vacuum system, a condensate is aqueous phenol, and the aqueous phenol enters an ethylbenzene tank (526); and tower bottoms are a bisphenol A phenol solution, and are pumped to a sprayed phenol cooler (308) for cooling through a phenol circulating pump (307), and then returns to the tower overhead of the phenol absorption tower (306).

According to the mother solution recycling unit (400#): a material from a mother solution receiving tank (401) is sequentially pumped to first-stage feed preheaters (403) and second-stage feed preheater (404) through a falling film evaporator feed pump (402); the first-stage feed preheater (403) is a thermo-coupled heat exchanger, and a heating medium is a gaseous phase material from a third-stage gas-liquid separation tank (411); the second-stage feed preheater (404) is also a thermo-coupled heat exchanger, and a heating medium is the phenol from a phenol recycling pump (419); the material is divided into two parts by weight after being two-stage preheated, one part of the material enters a high-pressure falling film evaporator (406) and is vaporized under the heating of the water vapor, and a gas-liquid two-phase material flowing out of a lower portion of the high-pressure falling film evaporator (406) enters a first-stage gas-liquid separation tank (407); the liquid phase material is pumped to a third-stage gas-liquid separation tank (411) through a first-stage separation tank discharge pump (410), the gaseous phase material is used as a heating medium, and enters a low-pressure falling film evaporator (405), and the condensed gaseous phase is separated from the condensed liquid phase in a second-stage gas-liquid separation tank (408); the liquid phase enters the phenol recycling tank (416), the gaseous phase enters a tail gas condenser (409) and is further condensed through the circulating water, a condensate enters the phenol recycling tank (416), and tail gas is delivered to the vacuum system; the other part of the preheated mother solution is heated and vaporized in the low-pressure falling film evaporator (405), and a gaseous phase material and a liquid phase material flowing out of a lower portion of the low-pressure falling film evaporator (405) enters the third-stage gas-liquid separation tank (411); one part of the gaseous phase material, serving as a heating medium, enters the first-stage feed preheater (403), the redundant gaseous phase material is condensed in a condenser (414) under the action of the circulating water, tail gas of the first-stage feed preheater (403) and tail gas of the condenser (414) converge and then enter an aftercooler (415), the tail gas is further condensed under the action of the circulating water and returns to the vacuum system, and a condensate of the first-stage feed preheater (403), a condensate of the condenser (414) and a condensate of the aftercooler (415) automatically flow to the phenol recycling tank (416); the material in the phenol recycling tank (416) is pumped into a phenol recycling storage tank (418) through a phenol discharge pump (417), then pumped out through the phenol recycling pump (419), and delivered to an outlet of the first-stage cooler (101) of the condensation reactor and an outlet of the phenol circulating pump (307) respectively after being cooled down through heat exchange by the second-stage feed preheater (404) and the cracking feed preheater (601); the liquid phase material in the third-stage gas-liquid separation tank (411) is a concentrated bisphenol A phenol solution, is pumped out by a crystallizer feed pump (412) and cooled down by the circulating water through a crystallizer feed cooler (413), and then enters a recycling crystallizer (420); adduct slurry is pumped out of the recycling crystallizer (420) by a crystallization circulating pump (422) and then enters a crystallization cooler (421), and is cooled down by deionized water to remove heat of crystallization from the slurry; the slurry flowing out of the recycling crystallizer (420) is continuously delivered to a secondary pressurized separating unit (423) for solid-liquid separation; the phenol used to wash the filter cake is the second-stage centrifuge filtrate from the slurry mixing washing mother solution pump (214); the mother solution separated from the secondary pressurized separating unit (423) enters a mother solution receiving tank (429) and is pumped out by a mother solution discharge pump (430), and is preheated by a mother solution heater (431), one part of the preheated mother solution is delivered to an isomerization reactor (432) for converting 2,4-bisphenol A, triphenol, chroman and the like therein into bisphenol A, and then the bisphenol A enters the mother solution receiving tank (401); the other part of the preheated mother solution is delivered to the cracking and rearrangement unit 600# for cracking and rearrangement; a washing solution from the secondary pressurized separating unit (423) enters a washing solution receiving tank (424) and is pumped to the mother solution receiving tank (401) through a washing solution discharge pump (425); one part of the metered reaction liquid from the condensation reaction concentrated solution discharge pump (121) is introduced from the primary separating unit, and mixed with the adduct crystals that have been filtered in a melt crystallizer (409); the material in the melt crystallizer (409) is pumped to a heater (428) by a corresponding melt crystallizer circulating pump (427) to be heated, and then returns to the melt crystallizer (409); and the material in the melt crystallizer (409) is pumped to the crystallization feed buffer tank (201) through the melt crystallizer circulating pump (427).

According to the solvent recycling unit (500#), a dehydration tower (502) is an azeotropic rectification tower, and ethylbenzene is an entrainer; the material from the light component discharge pump (125) is mixed with a material from an ethylbenzene feed pump (527), and the mixture enters the dehydration tower (502) after being preheated by low-pressure vapor in a dehydration tower feed preheater (501); gas from a tower overhead directly enters an acetone recycling tower (504), tower bottoms are ethylbenzene-containing phenol, and the tower bottoms are delivered to a phenol recycling tower (519) through a tower kettle discharge pump (518); gas from a tower overhead of the acetone recycling tower (504) is condensed through an acetone tower condenser (506) and an aftercooler (507), and tail gas enters the vacuum system; a condensate enters an acetone tower reflux tank (508) and is pumped out by an acetone tower reflux pump (509), one stream is delivered to the tower overhead of the acetone recycling tower to be used as reflux, and the other stream is used as recycled acetone and is delivered to an inlet of the second-stage feed pump (110) of the reactor in the condensation reaction unit 100#; a material in a tower kettle enters a phase splitter (510) to form a water phase and an organic phase; the water phase material is pumped out by a water phase discharge pump (511) and cooled down by coolers (512) and (513), and then enters a phase splitter (514) for further phase splitting; the organic phase automatically flows to the phase splitter (510), and the water phase is used as effluent of the device to be pumped out of a boundary region by an effluent pump (516) for biochemical treatment; the organic phase of the phase splitter (510) is pumped out by an ethylbenzene discharge pump (515), one part of the organic phase returns to the tower overhead of the dehydration tower (502) as reflux, and the other part of the organic phase is cooled down by an ethylbenzene cooler (517) and then enters a vacuum pump as a supplementary working liquid of an ethylbenzene liquid ring vacuum pump; the amount of rising vapor in the tower is controlled by adjusting the flow of heated vapor of a acetone tower reboiler (505); a material from the tower kettle discharge pump (518) of the dehydration tower (502) enters the phenol recycling tower (519); gas from the tower overhead is condensed through a phenol tower condenser (522) and a phenol tower aftercooler (523), tail gas enters the vacuum system, and a condensate enters a phenol tower reflux tank (524); one part of the condensate is used as reflux and returns to a tower overhead through a reflux pump (525) of the phenol recycling tower, the remaining condensate overflows to an ethylbenzene tank (526), the ethylbenzene tank (526) also receives aqueous phenol from the phenol absorption tower condenser (309), displaced ethylbenzene from the vacuum system and supplemented ethylbenzene, and the material in the ethylbenzene tank (526) is pumped to the dehydration tower (502) for feeding through the ethylbenzene feed pump (527); a tower kettle contains the ethylbenzene-free phenol, and the phenol is pumped to the phenol recycling storage tank (416) through a tower kettle discharge pump (521) of the phenol recycling tower; and the amount of rising vapor in the tower is controlled by adjusting the flow of the heated vapor of a phenol tower reboiler (520).

According to the cracking and rearrangement unit (600#), the mother solution from the mother solution discharge pump (430) enters a falling film evaporator (602) after being preheated by the cracking feed preheater (601); the cracking feed preheater (601) is a thermo-coupled heat exchanger, and a heating medium is recycled phenol from the second-stage feed preheater (404); the mother solution is vaporized in the falling film evaporator (602) under the heating of water vapor, a gaseous phase material and a liquid phase material enter a gas-liquid separation tank (603), the liquid phase enters a tower kettle of a cracking reactor (610), and the gaseous phase enters a de-p-isopropyl phenol tower (604); tower bottoms of the de-p-isopropyl phenol tower (604) are a heavy component containing p-isopropyl phenol, and are discharged out of the system through a discharge pump (606) for incineration treatment; gas from a tower overhead is condensed and cooled down by a condenser (607), tail gas enters the vacuum system, a condensate enters a reflux tank (608), and is pumped out by a reflux pump (609), one part of the condensate returns to the tower overhead of the de-p-isopropyl phenol tower (604) as reflux, and the other part of the condensate enters a tower overhead of the cracking reactor (610) as reflux of the cracking reactor (610); a material overflowing from the reflux tank (608) enters a cracking product tank (614); the amount of rising vapor in the tower is controlled by adjusting the flow of the heated water vapor of a reboiler (605); after the material from the gas-liquid separation tank (603) enters the tower kettle of the cracking reactor (610), the material is pumped to a cracking reaction heater (612) through a cracking reaction circulating pump (611) to be heated, a certain amount of alkali liquor is added as a catalyst of the cracking reaction, and the reactant material returns to the tower kettle of the cracking reactor (610); tar generated by cracking is discharged out of the system through the cracking reaction circulating pump (611) and is incinerated; gas from the tower overhead of the cracking reactor (610) is condensed and cooled through a cracking product condenser (613), tail gas enters the vacuum system, a condensate enters the cracking product tank (614), and is then pumped into a circulating cooler (616) for cooling through a rearrangement reaction feed pump (615); one part of the condensate circulates back to the cracking product tank (614), and the other part of the condensate is fed into a rearrangement reactor (617); and the rearranged material returns to the mother solution receiving tank (401).

A device for realizing a production process (1) for synthesizing bisphenol A is as follows: an outlet of the cracking feed preheater (601) at a cooling side is connected with an inlet of the first-stage cooler (101) at a cooling side after converging with an outlet of the first-stage circulating pump (105), an outlet of the first-stage cooler (101) at the cooling side is connected with an inlet of the first-stage reactor (102), an outlet of the first-stage reactor (102) is connected with an inlet of the first-stage reactor (103), an outlet of the first-stage reactor (103) is connected with an inlet of the first-stage reactor (104), an outlet of the first-stage reactor (104) is divided into two streams, which are respectively connected with an inlet of the first-stage circulating pump (105) and an inlet of the dehydration flash heater (106) at a heating side, an outlet of the dehydration flash heater (106) at the heating side is connected with an inlet of the dehydration flash tank (107), a gaseous phase outlet of the dehydration flash tank (107) is connected with an inlet of the first flash condenser (108) at a cooling side, a liquid phase outlet is connected with an inlet of the second-stage feed pump (110) after converging with an outlet of the acetone tower reflux pump (509), an outlet of the second-stage feed pump (110) is connected with an inlet of the second-stage cooler (111) at a cooling side, an outlet of the second-stage cooler (111) at the cooling side is connected with an inlet of the second-stage reactor (112), an outlet of the second stage reactor (112) is connected with an inlet of the second-stage reactor (113), an outlet of the second-stage reactor (113) is connected with an inlet of the second-stage reactor (114), a gaseous phase outlet of the first flash condenser (108) at a cooling side is connected with an inlet of the second flash condenser (109) at a cooling side, a liquid phase outlet of the first flash condenser (108) is connected with an inlet of the light component receiving tank (124) after converging with a liquid phase outlet of the second flash condenser (109) at the cooling side, a gaseous phase outlet of the second flash condenser (109) at the cooling side is connected with a vacuum unit, an outlet of the second-stage reactor (114) is connected with an inlet of an adsorption column (115) after converging with an outlet of the phenol circulating pump (307), an outlet of the adsorption column (115) is connected with an inlet of a de-light component tower feed preheater (116) at a heating side, an outlet of the de-light component tower feed preheater (116) at the heating side is connected with an inlet of the de-light component tower (117), a tower kettle outlet of the de-light component tower (117) is connected with an inlet of the tower kettle discharge pump (119) of the de-light component tower of a tower kettle of the de-light component tower, an outlet of the tower kettle discharge pump (119) of the tower kettle of the de-light component tower is connected with an inlet of the de-light component tower feed preheater (116) at a cooling side, an outlet of the de-light-component tower feed preheater (116) at the cooling side is connected with an inlet of the condensation reaction liquid receiving tank (120), an outlet of the condensation reaction liquid receiving tank (120) is connected with an inlet of the condensation reaction liquid discharge pump (121), an outlet of the condensation reaction liquid discharge pump (121) is divided into two streams, which are respectively connected with a filter cake outlet of the secondary pressurized separating unit (423) and an inlet of the crystallization feed buffer tank (201), a tower overhead outlet of the de-light component tower (117) is connected with an inlet of the de-light component tower condenser (122) at a cooling side, a gaseous phase outlet of the de-light component tower condenser (122) at the cooling side is connected with an inlet of the de-light component tower aftercooler (123) at a cooling side, a gaseous phase outlet of the de-light component tower aftercooler (123) at the cooling side is connected with the vacuum unit, and a liquid phase inlet of de-light component tower condenser (122) at the cooling side is connected with an inlet of the light component receiving tank (124) after converging with a liquid phase outlet of the de-light component tower aftercooler (123) at the cooling side.

A device for realizing a production process (2) for synthesizing bisphenol A is as follows: an outlet of the condensation reaction liquid discharge pump (121) is connected with an inlet of the crystallization feed buffer tank (201) after converging with an outlet of the melt crystallizer circulating pump (427), an outlet of the crystallization feed buffer tank (201) is connected with an inlet of the primary crystallizer feed pump (202), an outlet of the primary crystallizer feed pump (202) is connected with an inlet of the primary crystallizer feed cooler (203) at a cooling side, an outlet of the primary crystallizer feed cooler (203) at the cooling side and an outlet of the external circulation crystallization cooler (206) at a cooling side are both connected with an inlet of the primary crystallization circulator (204), an outlet of the primary crystallization circulator (204) is respectively connected with an inlet of the crystallization circulating pump (205) and a mother solution inlet of the first-stage primary pressurized separating unit (207), an outlet of the crystallization circulating pump (205) is connected with an inlet of the external circulation crystallization cooler (206) at the cooling side, a mother solution/washing solution outlet of the first-stage primary pressurized separating unit (207) is connected with an inlet of the mother solution/washing solution tank (208) of the first-stage primary separating unit, an outlet of the mother solution/washing solution tank (208) of the first-stage primary separating unit is connected with an inlet of the phenol washing pump (209), an outlet of the phenol washing pump (209) is connected with an inlet of the mother solution receiving tank (401), an outlet of the phenol discharge pump (311) is sequentially connected with a filter cake outlet of the first-stage primary pressurized separating unit (207) and an inlet of the slurry mixing washing tank (210), an outlet of the slurry mixing washing tank (210) is connected with an inlet of the slurry pump (211), an outlet of the slurry pump (211) is connected with a mother solution inlet of the second-stage primary pressurized separating unit (212), a mother solution outlet of the second-stage primary pressurized separating unit (212) is connected with an inlet of a mother solution tank (213) of the second-stage primary separating unit, an outlet of the mother solution tank (213) of the second-stage primary separating unit is connected with an inlet of the slurry mixing washing mother solution pump (214), an outlet of the slurry mixing washing mother solution pump (214) is divided into two streams, which are respectively connected with a washing solution inlet of the secondary pressurized separating unit (423) and a washing solution inlet of the first-stage primary pressurized separating unit (207), a filter cake outlet of the mother solution tank (213) of the second-stage primary separating unit and an outlet of the melt heater (217) at a heating side are both connected with an inlet of the melt crystallizer (215), an outlet of the melt crystallizer (215) is respectively connected with an inlet of the melt crystallizer circulating pump (216) and an inlet of the dephenolization feed pump (218), an outlet of the melt crystallizer circulating pump (216) is connected with an inlet of the melt heater (217) at the heating side, and an outlet of the dephenolization feed pump (218) is connected with an inlet of the falling film dephenolizer (301).

A device for realizing a production process (3) for synthesizing bisphenol A is as follows: an outlet of the dephenolization feed pump (218) is connected with an inlet of the falling film dephenolizer (301), an outlet of the falling film dephenolizer (301) is connected with an inlet of the gas-liquid separation tank (302), a gaseous phase outlet of the gas-liquid separation tank (302) is connected with an inlet of the stripper dephenolization tower condenser (303) at a cooling side, a gaseous phase outlet of the stripper dephenolization tower condenser (303) is connected with the vacuum unit, a liquid phase outlet is connected with an inlet of the phenol tank (310), an outlet of the phenol tank (310) is connected with an inlet of the phenol discharge pump (311), an outlet of the phenol discharge pump (311) is connected with an inlet of the phenol cooler (312) at a cooling side, a liquid phase outlet of the gas-liquid separation tank (302) is connected with a tower overhead inlet of the stripper dephenolization tower (305), a tower overhead outlet of the stripper dephenolization tower (305) is connected with a tower kettle inlet of the phenol absorption tower (306), a tower kettle outlet is connected with an inlet of the BPA melt pump (315), a tower kettle inlet is connected with an outlet of a stripper vapor superheater (304) at a heating side, an outlet of the BPA melt pump (315) is connected with an inlet of the packaging machine (316), a tower overhead outlet of the phenol absorption tower (306) is connected with an inlet of the phenol absorption tower condenser (309) at a cooling side, a tower kettle outlet is connected with an inlet of the phenol circulating pump (307), a tower overhead inlet is connected with an outlet of a sprayed phenol cooler (308) at a cooling side, a gaseous phase outlet of the phenol absorption tower condenser (309) at the cooling side is connected with the vacuum unit, a liquid phase outlet is connected with an inlet of the ethylbenzene tank (526), an outlet of the phenol circulating pump (307) is divided into streams, one stream is connected with an inlet of the absorption column (115) after converging with an outlet of the second-stage reactor (114), the other stream is connected with an inlet of the sprayed phenol cooler (308) at the cooling side after converging with a stream at an outlet of the cracking feed preheater (601) at a cooling side, an outlet of the fresh phenol tank (313) is connected with an inlet of the fresh phenol pump (314), an outlet of the fresh phenol pump (314) is divided into two streams, one stream is connected with an inlet of the phenol recycling tank (416), and the other stream is connected with a filter cake outlet of the first-stage primary pressurized separating unit (207) after converging with an outlet of the phenol cooler (312) at the cooling side.

A device for realizing a production process (4) for synthesizing bisphenol A is as follows: an inlet of the mother solution receiving tank (401) is respectively connected with an outlet of the phenol washing pump (209), an outlet of the rearrangement reactor (617), an outlet of the washing solution discharge pump (425) and an outlet of the isomerization reactor (432), an outlet of the mother solution receiving tank (401) is connected with an inlet of the falling film evaporator feed pump (402), the falling film evaporator feed pump (402) is connected with an inlet of the first-stage feed preheater (403) at a heating side, an outlet of the first-stage feed preheater (403) at the heating side is connected with an inlet of the second-stage feed preheater (404) at a heating side, an outlet of the second-stage feed preheater (404) at the heating side is divided into two streams, which are respectively connected with an inlet of the low-pressure falling film evaporator (405) at a heating side and an inlet of the pressurized falling film evaporator (406) at a heating side, an outlet of the pressurized falling film evaporator (406) at the heating side is connected with an inlet of the first-stage gas-liquid separation tank (407), a gaseous phase outlet of the first-stage gas-liquid separation tank (407) is connected with an inlet of the low-pressure falling film evaporator (405) at a cooling side, a liquid phase outlet is connected with an inlet of the first-stage separation tank discharge pump (410), an outlet of the first-stage separation tank discharge tank (410) is connected with an inlet of the third-stage gas-liquid separation tank (411), an outlet of the low-pressure falling film evaporator (405) at the heating side is connected with an inlet of the third-stage gas-liquid separation tank (411), an outlet of the low-pressure falling film evaporator at the cooling side is connected with an inlet of the second-stage gas-liquid separating tank (408), a gaseous phase outlet of the second-stage gas-liquid separating tank (408) is connected with an inlet of the tail gas condenser (409), a liquid phase outlet is connected with an inlet of the phenol recycling tank (416) after converging with a liquid phase outlet of the tail gas condenser (409), a liquid phase outlet of the third-stage gas-liquid separation tank (411) is connected with an inlet of the crystallizer feed pump (412), a gaseous phase outlet is divided into two streams, which are respectively connected with an inlet of the first-stage feed preheater (403) at a cooling side and an inlet of the condenser (414) at a cooling side, a gaseous phase outlet of the first-stage feed preheater (403) at the cooling side is connected with an inlet of the aftercooler (415) at a cooling side after converging with a gaseous phase outlet of the condenser (414) at the cooling side, a liquid phase inlet of the first-stage feed preheater (403) at the cooling side, a liquid phase inlet of the condenser (414) at the cooling side and a liquid phase inlet of the aftercooler (415) at the cooling side converge to be connected with the inlet of the phenol recycling tank (416), an outlet of the crystallizer feed pump (412) is connected with an inlet of the crystallizer feed cooler (413) at a cooling side, an outlet of the fresh phenol pump (313) and an outlet of the tower kettle discharge pump (521) of the phenol recycling tower are both connected with the inlet of the phenol recycling tank (416), an outlet of the phenol recycling tank (416) is connected with an inlet of the phenol recycling storage tank (418), an outlet of the phenol recycling storage tank (418) is connected with an inlet of the phenol recycling pump (419), an outlet of the phenol recycling pump (419) is connected with an inlet of the second-stage feed preheater (404) at a cooling side, an outlet of the second-stage feed preheater (404) at the cooling side is connected with an inlet of the cracking feed preheater (601) at a cooling side, an inlet of the recycling crystallizer (420) is respectively connected with an outlet of the crystallizer feed cooler (413) at the cooling side and an outlet of the external circulation crystallization cooler (421) at a cooling side, an outlet of the recycling crystallizer is respectively connected with a mother solution inlet of the crystallization circulating pump (422) and a mother solution inlet of the secondary pressurized separating unit (423), an outlet of the crystallization circulating pump (422) is connected with an inlet of the external circulation crystallization cooler (421) at the cooling side, an outlet of the slurry mixing washing mother solution pump (214) is connected with a washing solution inlet of the secondary pressurized separating unit (423), a mother solution outlet of the secondary pressurized separating unit (423) is connected with an inlet of the mother solution receiving tank (429), an outlet of the mother solution receiving tank (429) is connected with an inlet of the mother solution discharge pump (430), an outlet of the mother solution discharge pump (430) is divided into two streams, one stream is connected with an inlet of the mother solution heater (431) at a heating side, the other stream is further divided into two streams, which are respectively connected with an inlet of the isomerization reactor (432) and an inlet of the cracking feed preheater (601) at a heating side, a washing solution outlet of the secondary pressurized separating unit (423) is connected with an inlet of the washing solution receiving tank (424), a filter cake outlet is respectively connected with an outlet of the condensation reaction liquid discharge pump (121) and an inlet of the melt crystallizer (426), an outlet of the melt crystallizer (426) is connected with an inlet of melt crystallizer circulating pump (427), an outlet of the melt crystallizer circulating pump (427) is divided into two streams, which are respectively connected with an inlet of the crystallization feed buffer tank (201) and an inlet of the melt crystallizer heater (428) at a heating side, and an outlet of the melt crystallizer heater (428) at the heating side is connected with an inlet of the melt crystallizer (426).

A device for realizing a production process (5) for synthesizing bisphenol A is as follows: an outlet of the light component discharge pump (125) is connected with an inlet of the dehydration tower preheater (501) at a heating side after converging with an outlet of the ethylbenzene feed pump (527), an outlet of the dehydration tower preheater (501) at the heating side is connected with an inlet of the dehydration tower (502), a tower overhead outlet of the dehydration tower (502) is connected with an inlet of the acetone recycling tower (504), a tower overhead inlet is connected with an outlet of the ethylbenzene discharge pump (515), a tower kettle outlet is connected with an inlet of the tower kettle discharge pump (518) of the dehydration tower, a tower overhead outlet of the acetone recycling tower (504) is connected with an inlet of the acetone tower condenser (506) at a cooling side, a tower overhead inlet is connected with an outlet of the acetone tower reflux pump (509), a tower kettle outlet is connected with an inlet of the phase splitter (510), a gaseous phase outlet of the acetone tower condenser (506) at the cooling side is connected with an inlet of the acetone tower aftercooler (507) at a cooling side, a liquid phase outlet is connected with an inlet of the acetone tower reflux tank (508), a gaseous phase outlet of the acetone tower aftercooler (507) at the cooling side is connected with the vacuum unit, a liquid phase outlet is connected with the inlet of the acetone tower reflux tank (508), an outlet of the acetone tower reflux tank (508) is connected with an inlet of the acetone tower reflux pump (509), the outlet of the acetone tower reflux pump (509) is further connected with an inlet of the second-stage feed pump (110), an outlet of the phase splitter (510) is respectively connected with an inlet of the water phase discharge pump (511) and an inlet of the ethylbenzene discharge pump (515), an outlet of the water phase discharge pump (511) is connected with an inlet of the phenol-containing water cooler (512) at a cooling side, an outlet of the phenol-containing water cooler (512) at the cooling side is connected with the inlet of the phenol-containing water cooler (513) at the cooling side, an outlet of the phenol-containing water cooler (513) at the cooling side is connected with an inlet of the phase splitter (514), an outlet of the phase splitter (514) is respectively connected with the inlet of the phase splitter (510) and an inlet of the effluent pump (516), an outlet of the ethylbenzene discharge pump (515) is further connected with an inlet of the ethylbenzene cooler (517) at a cooling side, an outlet of the ethylbenzene cooler (517) at the cooling side is connected with the vacuum unit, an outlet of the tower kettle discharge pump (518) of the dehydration tower is connected with an inlet of the phenol recycling tower (519), a tower overhead outlet of the phenol recycling tower (519) is connected with an inlet of the phenol tower condenser (522) at a cooling side, a tower overhead inlet is connected with an outlet of the reflux pump (525) of the phenol recycling tower, a tower kettle outlet is connected with an inlet of the tower kettle discharge pump (521) of the phenol recycling tower, a gaseous phase outlet of the phenol tower condenser (522) at the cooling side is connected with an inlet of the phenol tower aftercooler (523) at a cooling side, a liquid phase outlet is connected with an inlet of the phenol tower reflux tank (524), a gaseous phase outlet of the phenol tower aftercooler (523) at the cooling side is connected with the vacuum unit, and a liquid phase outlet is connected with the inlet of the phenol tower reflux tank (524).

A device for realizing a production process (6) for synthesizing bisphenol A is as follows: an outlet of the mother solution discharge pump (430) is connected with an inlet of the cracking feed preheater (601) at a heating side, an outlet of the cracking feed preheater (601) at the heating side is connected with an inlet of the falling film evaporator (602), an outlet of the falling film evaporator (602) is connected with an inlet of the gas-liquid separation tank (603), a gaseous phase outlet of the gas-liquid separation tank (603) is connected with an inlet of the de-p-isopropyl phenol tower (604), a liquid phase outlet is connected with an inlet of the cracking reactor (610), a tower overhead outlet of the de-p-isopropyl phenol tower (604) is connected with an inlet of the condenser (607) at a cooling side, a tower kettle outlet is connected with an inlet of the tower kettle discharge pump (606), an outlet of the reflux pump (609) is divided into three streams, which are respectively connected with a tower overhead inlet of the de-p-isopropyl phenol tower (604), an inlet of the cracking product tank (614), and a tower overhead inlet of the cracking reactor (610), a gaseous phase outlet of the condenser (607) at the cooling side is connected with the vacuum unit, a liquid phase outlet is connected with an inlet of the reflux tank (608), an outlet of the reflux tank (608) is connected with an inlet of the reflux pump (609), a tower overhead outlet of the cracking reactor (610) is connected with an inlet of the cracking product condenser (613) at a cooling side, a tower kettle outlet is connected with an inlet of the cracking reaction circulating pump (611), an outlet of the cracking reaction circulating pump (611) is divided into two streams, which are respectively connected with an inlet of the circulating heater (612) at a heating side and an incinerator, an outlet of the circulating heater (612) at the heating side is connected with a tower kettle inlet of the cracking reactor (610) after converging with a basic catalyst, a gaseous phase outlet of the cracking product condenser (613) at the cooling side is connected with the vacuum unit, a liquid phase outlet of the cracking product condenser (613) is connected with an inlet of the cracking product tank (614), an outlet of the cracking product tank (614) is connected with an inlet of the rearrangement feed pump (615), the inlet of the rearrangement feed pump (615) is connected with an inlet of the circulating cooler (616) at a cooling side, an outlet of the circulating cooler (616) at the cooling side is divided into two streams, which are respectively connected with the inlet of the cracking product tank (614) and an inlet of the rearrangement reactor (617), and an outlet of the rearrangement reactor (617) is connected with an inlet of the mother solution recycling tank (401).

A temperature at the outlet of the first-stage cooler (101) at the cooling side is 65-70°C, a temperature at the outlet of the preheater (106) at the heating side is 85-87°C, an operating pressure of the dehydration flash tank (107) is 6-8 kPaA, a temperature at the outlet of the second-stage cooler (111) at the cooling side is 70-75°C, an operating pressure of the first-stage primary pressurized separating unit (207) is 300-550 kPaA, an operating pressure of the second-stage primary pressurized separating unit (212) is 350-600 kPaA, an operating temperature of the high-pressure falling film evaporator (406) is 150-154°C, an operating pressure is 33-35 kPaA, an operating temperature of the low-pressure falling film evaporator (405) is 112-114°C, an operating pressure is 6-8 kPaA, and a temperature at the inlet of the isomerization reactor (432) is 70-75°C.

The present invention relates to a production process and device for synthesizing bisphenol A by a resin method. The production device includes a condensation reaction unit, a first-stage adduct crystallization unit, a liquid-phase dephenolization unit, a secondary adduct crystallization unit, a solvent recycling unit, and a cracking and rearrangement unit, wherein the condensation reaction unit is configured to perform a catalytic condensation reaction between phenol and acetone to generate bisphenol A in the presence of a resin catalyst; the first-stage adduct crystallization unit is configured to mix a concentrated solution from the condensation reaction with adduct crystals recycled after melting and then perform adduct crystallization; the liquid-phase dephenolization unit is configured to melt an adduct to yield a bisphenol A product; the secondary adduct crystallization unit is configured to recycle phenol and bisphenol A from a mother solution; the solvent recycling unit is configured to recycle unreacted phenol and unreacted acetone, and discharge phenol-containing process water for sewage treatment; and the cracking and rearrangement unit is configured to recycle the phenol from the mother solution, and convert bisphenol A, 2,4-bisphenol A and the like in the mother solution into the bisphenol A that returns to a system through cracking and rearrangement. In order to improve the yield and purity of the phenol and reduce energy consumption, the steps of the external circulation of the first-stage reactors, flash evaporation dehydration between the two stages of reactors, solid-liquid separation of the high-pressure separating unit, double-effect falling film evaporation, conversion by the isomerization reactor and the like are introduced into a process flow, and the application of the steps ensures that the purity of the finally yielded bisphenol A product is more than 99.97wt%, the free phenol is less than 10 ppm, the 2, 4-bisphenol A is less than 60 ppm, the triphenol is less than 25 ppm, the water is less than 480 ppm, and the consumption of standard oil is less than 198 kg per ton of the bisphenol A product.

The present invention has the following advantages and beneficial effects:
1. the production process and device for synthesizing bisphenol A of the present invention have the advantages that a unique catalytic dehydration condensation reaction process is developed to realize the high single-pass conversion rate of acetone and the high selectivity of bisphenol A generation, a cascade two-section packed-bed flow is adopted, the first-stage reactors adopt external circulation for heat removal, the average temperatures in the reactors are reduced, the reverse reaction is inhibited, and the single-pass conversion rate is improved;
2. the production process and device for synthesizing bisphenol A of the present invention have the advantage that the water is removed in time from the front of the second-stage reactors through flash evaporation, so that the chemical balance inhibition effect of the water on the condensation reaction for generating bisphenol A and the activity inhibition effect on the resin catalyst for the condensation reaction are eliminated, and the higher single-pass conversion rate of the second-stage reactors is realized;
3. the production process and device for synthesizing bisphenol A of the present invention have the advantage that the pressurized drum centrifuge is used for solid-liquid two-phase separation in the primary and secondary flows, so that the separation driving force and the separation effect are substantially increased, and the difficulty and energy consumption for the phenol removal and phenol recovery of products in the subsequent section are reduced;
4. the production process and device for synthesizing bisphenol A of the present invention have the advantages that due to the application of a thermal coupling technology and a double-effect falling film evaporation technology, vapor consumption is reduced significantly, and the vapor consumption index is advanced; and
5. the production process and device for synthesizing bisphenol A of the present invention have the advantages that the isomerization reactor is adopted to convert byproducts into bisphenol A, the yield of the bisphenol A is increased, the proportion of heavy components to be incinerated is reduced, and environmental pollution is mitigated.

### BRIEF DESCRIPTION OF THE DRAWINGS

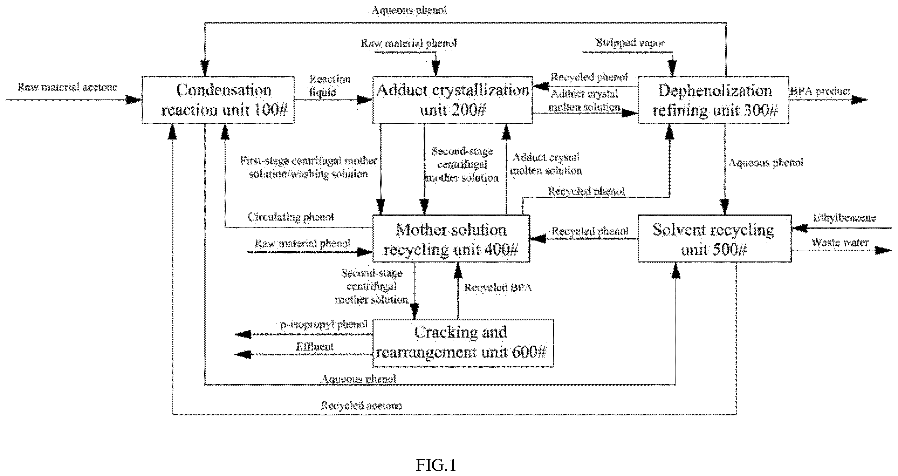

FIG. 1 is a flow block diagram of a process for synthesizing bisphenol A by a resin method;
FIG. 2 is a flowchart of a condensation reaction unit 100#;
FIG. 3 is a flowchart of a first-stage adduct crystallization unit 200#;
FIG. 4 is a flowchart of a liquid phase dephenolization unit 300#;
FIG. 5 is a flow chart of a secondary adduct crystallization unit 400;
FIG. 6 is a flowchart of a solvent recycling unit 500#;
FIG. 7 is a flow chart of a cracking and rearrangement unit 600#;

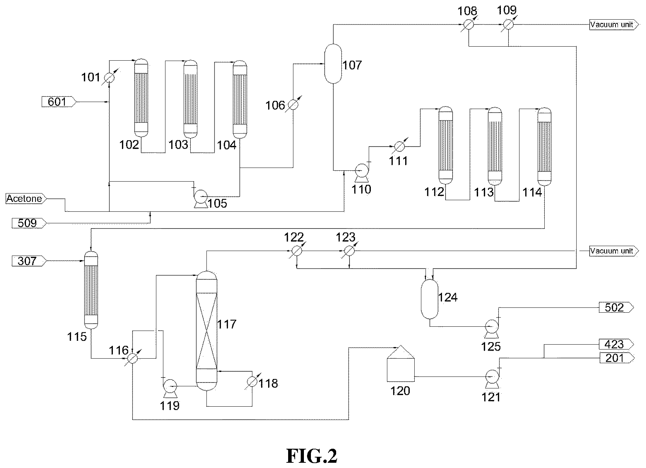

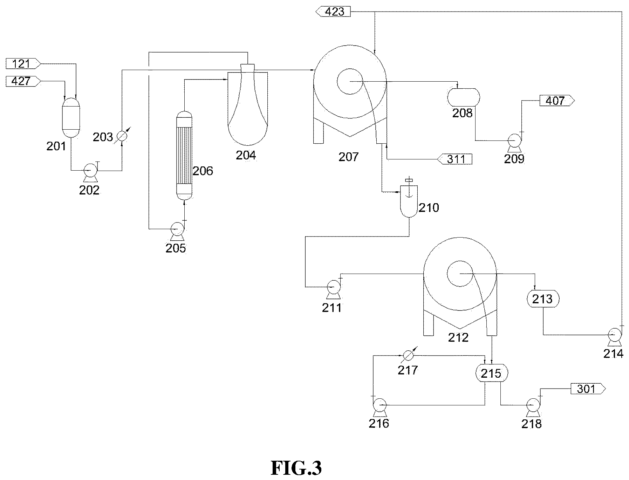

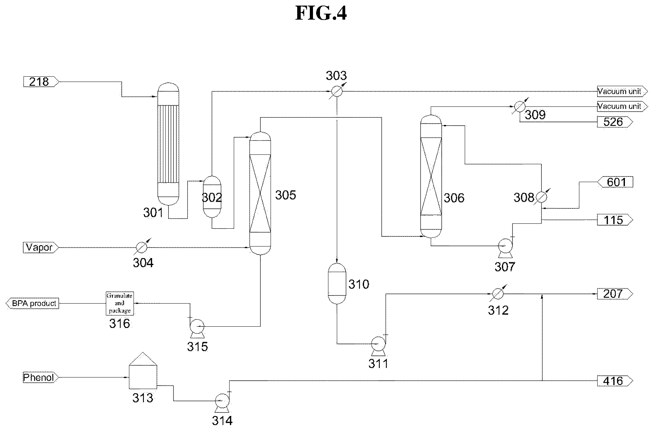

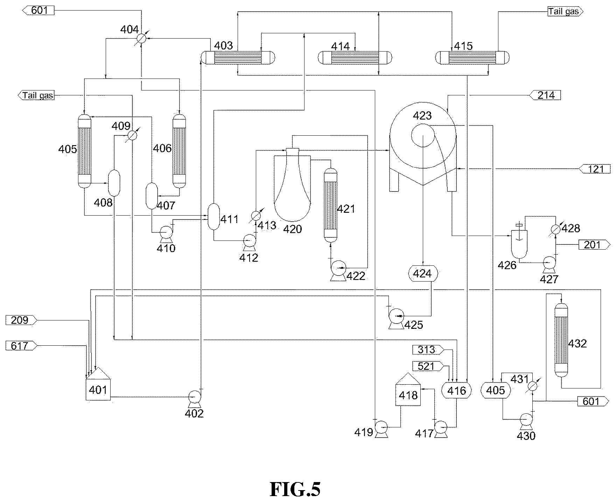

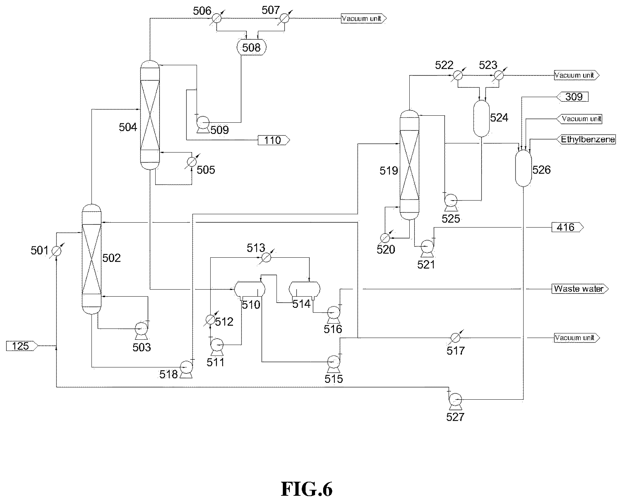

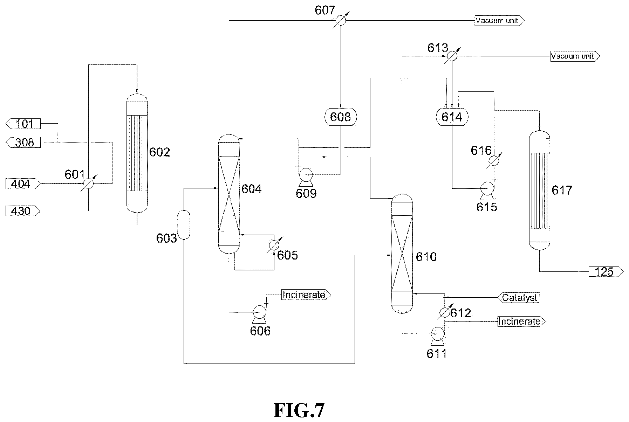

wherein: as shown in FIG. 2,101: first-stage cooler, 102: first-stage reactor, 103, 104: first-stage reactor, 105: first-stage circulating pump, 106: dehydration flash heater, 107: dehydration flash tank, 108: first flash condenser, 109: second flash condenser, 110: second-stage feed pump, 111: second-stage cooler, 112: second-stage reactor, 113: second-stage reactor, 114: second-stage reactor, 115: adsorption column, 116 : de-light component tower feed preheater, 117: de-light component tower, 118: de-light component tower reboiler, 119: tower kettle discharge pump of the de-light component tower, 120: condensation reaction liquid receiving tank, 121: condensation reaction liquid discharge pump, 122: de-light component tower condenser, 123: de-light component tower aftercooler, 124: light component receiving tank (124);
As shown in FIG.3, 201: crystallization feed buffer tank, 202: primary crystallizer feed pump, 203: primary crystallizer feed cooler, 204: primary crystallization circulator, 205: crystallization circulating pump, 206: external circulation crystallization cooler, 207: first-stage primary pressurized separating unit, 208: mother solution/washing solution tank of the first-stage primary separating unit, 209: phenol washing pump), 210: slurry mixing washing tank, 211: slurry pump, 212: second-stage primary pressurized separating unit, 213: mother solution tank of the second-stage primary separating unit, 214: slurry mixing washing mother solution pump, 215: melt crystallizer, 216: melt crystallizer circulating pump, 217: melt heater, 218: dephenolization feed pump;
As shown in FIG.4, 301: falling film dephenolizer, 302: gas-liquid separation tank, 303: stripper dephenolization tower condenser, 304: stripper vapor superheater, 305: stripper dephenolization tower, 306: phenol absorption tower, 307: phenol circulating pump, 308: sprayed phenol cooler, 309: phenol absorption tower condenser, 310: phenol tank, 311: phenol discharge pump, 312: phenol cooler, 313: fresh phenol tank, 314: fresh phenol pump, 315: BPA melt pump (315), 316: packaging machine;
As shown in FIG.5, 401: mother solution receiving tank, 402: falling film evaporator feed pump, 403: first-stage feed preheater, 404: second-stage feed preheater, 405: low-pressure falling film evaporator, 406: pressurized falling film evaporator, 407: first-stage gas-liquid separation tank, 408: second-stage gas-liquid separation tank, 409: tail gas condenser, 410: first-stage separation tank discharge pump, 411: third-stage gas-liquid separation tank, 412: crystallizer feed pump, 413: crystallizer feed cooler, 414: condenser, 415: aftercooler, 416: phenol recycling tank, 417: phenol discharge pump, 418: phenol recycling storage tank, 419: phenol recycling pump, 420: recycling crystallizer, 421 external circulation crystallization cooler, 422: crystallization circulating pump, 423: secondary pressurized separating unit, 424: washing solution receiving tank, 425: washing solution discharge pump, 426: melt crystallizer, 427: melt crystallizer circulating pump, 428: melt crystallizer heater, 429: mother solution receiving tank, 430: mother solution discharge pump, 431: mother solution heater, 432: isomerization reactor;
As shown in FIG.6, 501: dehydration tower preheater, 502: dehydration tower, 503: dehydration tower reboiler, 504: acetone recycling tower, 505: acetone tower reboiler, 506: acetone tower condenser, 507: acetone tower aftercooler, 508: acetone tower reflux tank, 509: acetone tower reflux pump, 510: phase splitter, 511: water phase discharge pump, 512: phenol-containing water cooler, 513: phenol-containing water cooler, 514: phase splitter, 515: ethylbenzene discharge pump, 516: effluent pump, 517: ethylbenzene cooler, 518: tower kettle discharge pump of the dehydration tower, 519: phenol recycling tower, 520: phenol tower reboiler, 521: tower kettle discharge pump of the phenol recycling tower, 522: phenol tower condenser, 523: phenol tower aftercooler, 524: phenol tower reflux tank, 525: reflux pump of the phenol recycling tower, 526: ethylbenzene tank, 527: ethylbenzene feed pump; and
As shown in FIG.7, 601: cracking feed preheater, 602: falling film evaporator, 603: gas-liquid separation tank, 604: de-p-isopropyl phenol tower, 605: reboiler, 606: tower kettle discharge pump, 607: condenser, 608: reflux tank, 609: reflux pump, 610: cracking reactor, 611: cracking reaction circulating pump, 612: circulating heater, 613: cracking product condenser, 614: cracking product tank (614), 615: rearrangement feed pump, 616: circulating cooler and 617: rearrangement reactor.

### DETAILED DESCRIPTION OF THE EMBODIMENTS

### Embodiment 1:

Refined fresh acetone, at flow of 16000 kg/h, is respectively fed into a first-stage reactor 102 and a second-stage reactor 112 by weight in proportion; the acetone fed into the first-stage reactor 102 is mixed with recycled phenol from a cracking feed preheater 601 and a material from a first-stage circulating pump 105, and a mixture enters a first-stage cooler 101, and enters the first-stage reactors 102-104 after being cooled to 65°C by circulating water; and one part of reaction liquid flowing out of the first-stage reactor 104 circulates back to the first-stage reactor 102 through the first-stage circulating pump 105, with circulating flow of 520000 kg/h, and the remaining reaction liquid is heated to 85°C by a preheater 106 and enters a dehydration flash tank 107 to remove part of water from the remaining reaction liquid, with an operating pressure of the flash tank of 6 kPaA. Flash gas from the dehydration flash tank 107 is condensed and cooled down by a first flash condenser 108 and a second flash condenser 109, and a condensate containing water, acetone and phenol automatically flows to a light component receiving tank 124, while tail gas is fed into a vacuum system; a material dehydrated from the dehydration flash tank 107 is mixed with the recycled acetone and a second stream of fresh acetone, and the mixed material is then fed into a second-stage cooler 111 through a second-stage feed pump 110, and enters second-stage reactors 112-114 after being cooled to 70°C by the circulating water; a condensation reaction liquid from the second-stage reactor 114 is mixed with tower bottoms from a phenol absorption tower 306, then a mixture enters an adsorption column 115, and passes through a preheater 116 to be heated by heat exchange with tower bottoms of a de-light component tower 117 before entering the de-light component tower 117; the water, the unreacted acetone and one part of the phenol in the reaction liquid are distilled out of a tower overhead, and after condensation through a condenser 122 and an aftercooler 123, tail gas enters the vacuum system, and a condensate automatically flows into the light component receiving tank 124 and is delivered to a solvent recycling unit 500# through a discharge pump 125 for recycling the phenol and the acetone; a de-light component tower reboiler 118 is configured to concentrate a material in a tower kettle through heating by low-pressure vapor, and the concentrated material is pumped out by a discharge pump 119, cooled down by heat exchange in the preheater 116, and delivered to a condensation reaction liquid receiving tank 120.

A reaction concentrated solution from a condensation reaction liquid discharge pump 121 and a recycled adduct crystal molten solution of a mother solution recycling unit 400# from a melt crystallizer circulating pump 427 are mixed in a crystallization feed buffer tank 201, then pumped out by a crystallizer feed pump 202 and cooled down by a feed cooler 203, and enters a primary crystallization circulator 204; slurry is pumped out of the primary crystallization circulator 204 by a crystallization circulating pump 205, and then enters a crystallization cooler 206, and heat of crystallization is removed from the slurry under a cooling effect of deionized water; the slurry flowing out of the primary crystallization circulator 204 is continuously delivered to a primary pressurized separating unit 207 for solid-liquid separation through a flow control valve; the phenol for washing a filter cake is a second-stage centrifuge filtrate from a slurry mixing washing mother solution pump 214, and the mother solution separated from the slurry and the washing solution enter a mother solution/washing solution tank 208 of the primary separating unit, and are pumped to a mother solution receiving tank 401 through a discharge pump 218; the phenol from a phenol discharge pump 311 is introduced from the primary separating unit, and and the phenol and the filtered adduct crystals undergo mixing and washing in a slurry mixing washing tank 210; the slurry undergoing slurry mixing is respectively pumped to a second-stage primary pressurized separating unit 212 for solid-liquid separation through a corresponding slurry pump 211; a filtrate enters a slurry mixing centrifuge mother solution tank 213, is pumped out by the slurry mixing washing mother solution pump 214 and is respectively delivered to the primary pressurized separating unit 207 and a secondary pressurized separating unit 423 for washing the phenol; the filter cake is removed by a centrifuge and fed into a melt crystallizer 215; a material in the melt crystallizer 215 is pumped to a melt heater 217 by a corresponding melt crystallizer circulating pump 216 to be heated, and then returns to the melt crystallizer 215. An operating pressure of the first-stage primary pressurized separating unit 207 and an operating pressure of the second-stage primary pressurized separating unit 212 are 300 kPaA and 350 kPaA, respectively.

An adduct molten solution from a dephenolization feed pump 218 enters a falling film dephenolizer 301, is vaporized under the heating of water vapor, and enters a gas-liquid separation tank 302; a liquid phase enters a tower overhead of a stripper dephenolizer 305; a gaseous phase is condensed by a condenser 303, tail gas enters the vacuum system, and a condensate enters a phenol tank 310; fresh phenol from a tank zone enters a fresh phenol tank 313 at flow of 48864 kg/h, and is pumped out by a fresh phenol pump 314, one part of the fresh phenol is fed into the phenol tank 310 after being metered, and the remaining fresh phenol is delivered to a phenol recycling tank 416; the phenol in the phenol tank 310 is pumped out by a phenol discharge pump 311, cooled down to a set value by a phenol washing cooler 312, and is then delivered to the slurry mixing washing tank 210 for washing; the water vapor enters a tower bottom of the stripper dephenolizer 305 as stripped vapor after being heated by an electrically heated vapor superheater 304; liquid bisphenol A yielded at the tower bottom is pumped out by a BPA melt pump 315 and then formed and granulated, and the granulated bisphenol A enters a packaging machine 316 for packaging, so as to yield a bisphenol A product; a gaseous phase at a tower overhead enters a tower bottom of a phenol absorption tower 306 and performs gas-liquid mass transfer with the absorbed phenol added from a tower overhead; the gas discharged from the tower overhead enters a condenser 309, tail gas enters the vacuum system, a condensate is aqueous phenol, and the aqueous phenol enters an ethylbenzene tank 526; and tower bottoms are a bisphenol A phenol solution, and are pumped to a cooler 308 for cooling through a phenol circulating pump 307, and then returns to the tower overhead of the phenol absorption tower 306.

A material from a mother solution receiving tank 401 is sequentially pumped to feed preheaters 403,404 through a feed pump 402; the first-stage feed preheater 403 is a thermo-coupled heat exchanger, and a heating medium is a gaseous phase material from a third-stage gas-liquid separation tank 411; the second-stage feed preheater 404 is also a thermo-coupled heat exchanger, and a heating medium is the phenol from a phenol recycling pump 419; the material is divided into two parts by weight in proportion after being two-stage preheated, one part of the material enters a high-pressure falling film evaporator 406 and is vaporized under the heating of the water vapor, a gas-liquid two-phase material flowing out of a lower portion of the high-pressure falling film evaporator 406 enters a gas-liquid separation tank 407, and an operating temperature and an operating pressure of the high-pressure falling film evaporator 406 are respectively 150°C and 33 kPaA; the liquid phase material is pumped to a third-stage gas-liquid separation tank 411 through a first-stage separation tank discharge pump 410, the gaseous phase material is used as a heating medium, and enters a low-pressure falling film evaporator 405, and the condensed gaseous phase is separated from the condensed liquid phase in a gas-liquid separation tank 408; the liquid phase enters the phenol recycling tank 416, the gaseous phase enters a tail gas condenser 409 and is further condensed through the circulating water, a condensate enters the phenol recycling tank 416, and tail gas is delivered to the vacuum system; the other part of the preheated mother solution is heated and vaporized in the low-pressure falling film evaporator 405, a gas-liquid two-phase material flowing out of a lower portion of the low-pressure falling film evaporator 405 enters the third-stage gas-liquid separation tank (11, and an operating temperature and an operating pressure of the low-pressure falling film evaporator 405 are respectively 112°C and 6 kPaA; one part of the gaseous phase material, serving as a heating medium, enters the first-stage feed preheater 403, the redundant gaseous phase material is condensed in a condenser 414 under the action of the circulating water, tail gas of the first-stage feed preheater 403 and tail gas of the condenser 414 converge and then enter an aftercooler 415, the tail gas is further condensed under the action of the circulating water and returns to the vacuum system, and a condensate of the first-stage feed preheater 403, a condensate of the condenser 414 and a condensate of the aftercooler 415 automatically flow to the phenol recycling tank 416; the material in the phenol recycling tank 416 is pumped into a phenol recycling storage tank 418 through a phenol discharge pump 417, then pumped out through the phenol recycling pump 419, and delivered to an outlet of a first-stage cooler 101 of the condensation reactor and an outlet of a phenol circulating pump 307 respectively after being cooled down through heat exchange by the second-stage feed preheater 404 and the cracking feed preheater 601; the liquid phase material in the third-stage gas-liquid separation tank 411 is a concentrated bisphenol A phenol solution, is pumped out by a crystallizer feed pump 412 and cooled down by the circulating water through a crystallizer feed cooler 413, and then enters a recycling crystallizer 420; adduct slurry is pumped out of the recycling crystallizer 420 by a crystallization circulating pump 422, enters a crystallization cooler 421, and is cooled down by deionized water to remove heat of crystallization from the slurry; the slurry flowing out of the recycling crystallizer 420 is continuously delivered to a secondary pressurized separating unit 423 for solid-liquid separation; the phenol used to wash the filter cake is the second-stage centrifuge filtrate from the slurry mixing washing mother solution pump 214; the mother solution separated from the secondary pressurized separating unit 423 enters a mother solution receiving tank 429, is pumped out by a mother solution discharge pump 430, and is preheated by a mother solution heater 431, one part of the preheated mother solution is delivered to an isomerization reactor 432 for converting 2, 4-bisphenol A, triphenol, chroman and the like therein into bisphenol A, with a temperature at an inlet being 70°C, and then the bisphenol A enters the mother solution receiving tank 401; the other part of the preheated mother solution is delivered to a cracking and rearrangement unit 600# for cracking and rearrangement; a washing solution from the secondary pressurized separating unit 423 enters a washing solution receiving tank 424 and is pumped to the mother solution receiving tank 401 through a washing solution discharge pump 425; one part of the metered reaction liquid from the condensation reaction concentrated solution discharge pump 121 is introduced from the primary separating unit, and mixed with the adduct crystals that have been filtered in a melt crystallizer 409; the material in the melt crystallizer 409 is pumped to a melt crystallizer heater 428 by the corresponding melt crystallizer circulating pump 427 to be heated, and then returns to the melt crystallizer 409; and the material in the melt crystallizer 409 is pumped to the crystallization feed buffer tank 201 through the melt crystallizer circulating pump 427.

A dehydration tower 502 is an azeotropic rectification tower, and ethylbenzene is an entrainer; the material from the light component discharge pump 121 is mixed with a material from an ethylbenzene feed pump 527, and the mixture enters the dehydration tower 502 after being preheated by low-pressure vapor in a dehydration tower feed preheater 501; gas from a tower overhead directly enters an acetone recycling tower 504, tower bottoms are ethylbenzene-containing phenol, and the tower bottoms are delivered to a phenol recycling tower 519 through a tower kettle discharge pump 518; gas from a tower overhead of the acetone recycling tower 504 is condensed through an acetone tower condenser (506) and an acetone tower aftercooler 507, and tail gas enters the vacuum system; a condensate enters an acetone tower reflux tank 508 and is pumped out by an acetone tower reflux pump 509, one stream is delivered to the tower overhead of the acetone recycling tower to be used as reflux, and the other stream is used as recycled acetone and is delivered to an inlet of the second-stage feed pump 110 of the reactor in a condensation reaction unit 100#; a material in a tower kettle enters a phase splitter 510 to form a water phase and an organic phase; the water phase material is pumped out by a water phase discharge pump 511 and cooled down by coolers 512,513, and then enters a phase splitter 514 for further phase splitting; the organic phase automatically flows to the phase splitter 510, and the water phase is used as effluent of the device to be pumped out of a boundary region by an effluent pump 516 for biochemical treatment; the organic phase of the phase splitter 510 is pumped out by an ethylbenzene discharge pump 515, one part of the organic phase returns to the tower overhead of the dehydration tower 502 as reflux, and the other part of the organic phase is cooled down by an ethylbenzene cooler 517 and then enters a vacuum pump as a supplementary working liquid of an ethylbenzene liquid ring vacuum pump; the amount of rising vapor in the tower is controlled by adjusting the flow of heated vapor of a reboiler 505; a material from the tower kettle discharge pump 518 of the dehydration tower 502 enters the phenol recycling tower 519; gas from the tower overhead is condensed through a phenol tower condenser 522 and a phenol tower aftercooler 523, tail gas enters the vacuum system, and a condensate enters a phenol tower reflux tank 524; one part of the condensate is used as reflux and returns to the tower overhead through a reflux pump 525 of the phenol recycling tower, the remaining condensate overflows to an ethylbenzene tank 526, the ethylbenzene tank 526 also receives aqueous phenol from the phenol absorption tower condenser 309, displaced ethylbenzene from the vacuum system and supplemented ethylbenzene, and the material in the ethylbenzene tank 526 is pumped to the dehydration tower 502 for feeding through the ethylbenzene feed pump 527; a tower kettle contains the ethylbenzene-free phenol, and the phenol is pumped to the phenol recycling storage tank 416 through a tower kettle discharge pump 521; and the amount of rising vapor in the tower is controlled by adjusting the flow of the heated vapor of a phenol tower reboiler 520.

The mother solution from the mother solution discharge pump 430 enters a falling film evaporator 602 after being preheated by the cracking feed preheater 601; the cracking feed preheater 601 is a thermo-coupled heat exchanger and a heating medium is recycled phenol from the second-stage feed preheater 404; the mother solution is vaporized in the falling film evaporator 602 under the heating of water vapor, a gaseous phase material and a liquid phase material enter a gas-liquid separation tank 603, the liquid phase enters a tower kettle of a cracking reactor 610, and the gaseous phase enters a de-p-isopropyl phenol tower 604; tower bottoms of the de-p-isopropyl phenol tower 604 are a heavy component containing p-isopropyl phenol, and are discharged out of the system through a discharge pump 606 for incineration treatment; gas from a tower overhead is condensed and cooled down by a condenser 607, tail gas enters the vacuum system, a condensate enters a reflux tank 608, and is pumped out by a reflux pump 609, one part of the condensate returns to the tower overhead of the de-p-isopropyl phenol tower 604 as reflux, and the other part of the condensate enters a tower overhead of the cracking reactor 610 as reflux of the cracking reactor 610; a material overflowing from the reflux tank 608 enters a cracking product tank 614; the amount of rising vapor in the tower is controlled in the tower by adjusting the flow of the heated water vapor of a reboiler 605; after the material from the gas-liquid separation tank 603 enters the tower kettle of the cracking reactor (610), the material is pumped to a cracking reaction heater 612 through a cracking reaction circulating pump 611 to be heated, a certain amount of alkali liquor is added as a catalyst of the cracking reaction, and the reactant material returns to the tower kettle of the cracking reactor 610; tar generated by cracking is discharged out of the system through the cracking reaction circulating pump 611 and is incinerated; gas from the tower overhead of the cracking reactor 610 is condensed and cooled through a cracking product condenser 613, tail gas enters the vacuum system, a condensate enters the cracking product tank 614, and is then pumped into a circulating cooler 616 for cooling through a rearrangement feed pump 615; one part of the condensate circulates back to the cracking product tank 614, and the other part of the condensate is fed into a rearrangement reactor 617; and the rearranged material returns to the mother solution receiving tank 401.

The purity of the finally yielded bisphenol A product is more than 99.97wt%, the free phenol is less than 10 ppm, the 2,4-bisphenol A is less than 60 ppm, the triphenol is less than 25 ppm, the water is less than 480 ppm, and the consumption of standard oil is less than 193 kg per ton of the bisphenol A product.

### Embodiment 2:

Refined fresh acetone, at flow of 15000 kg/h, is respectively fed into a first-stage reactor 102 and a second-stage reactor 112 by weight in proportion; the acetone fed into the first-stage reactor 102 is mixed with recycled phenol from a cracking feed preheater 601 and a material from a first-stage circulating pump 105, and a mixture enters a first-stage cooler 101, and enters the first-stage reactors 102-104 after being cooled to 67°C by circulating water; and one part of the reaction liquid flowing out of the first-stage reactor 104 circulates back to the first-stage reactor 102 through the first-stage circulating pump 105, with circulating flow of 487500 kg/h, and the remaining reaction liquid is heated to 86°C by a preheater 106 and enters a dehydration flash tank 107 to remove part of water from the remaining reaction liquid, with an operating pressure of the flash tank of 7 kPaA. Flash gas from the dehydration flash tank 107 is condensed and cooled down by a first flash condenser 108 and a second flash condenser 109, and a condensate containing water, acetone and phenol automatically flows to a light component receiving tank 124, while tail gas is fed into a vacuum system; a material dehydrated from the dehydration flash tank 107 is mixed with the recycled acetone and a second stream of fresh acetone, and the mixed material is then fed into a second-stage cooler 111 through a second-stage feed pump 110, and enters second-stage reactors 112-114 after being cooled to 72°C by the circulating water; a condensation reaction liquid from the second-stage reactor 114 is mixed with tower bottoms from a phenol absorption tower 306, then a mixture enters an adsorption column (115), and passes through a de-light component tower feed preheater 116 to be heated by heat exchange with tower bottoms of a de-light component tower 117 before entering the de-light component tower 117; the water, the unreacted acetone and one part of the phenol in the reaction liquid are distilled out of a tower overhead, and after condensation through a de-light component tower condenser 122 and a de-light component tower aftercooler 123, tail gas enters the vacuum system, and a condensate automatically flows into the light component receiving tank 124 and is delivered to a solvent recycling unit 500# through a discharge pump 125 for recycling the phenol and the acetone; a de-light component tower reboiler 118 is configured to concentrate a material in a tower kettle by heating low-pressure vapor, and the concentrated material is pumped out by a tower kettle discharge pump 119, cooled down by heat exchange in the de-light component tower feed preheater 116, and delivered to a condensation reaction liquid receiving tank 120.

A reaction concentrated solution from a condensation reaction liquid discharge pump 121 and a recycled adduct crystal molten solution of a mother solution recycling unit 400# from a melt crystallizer circulating pump 427 are mixed in a crystallization feed buffer tank 201, then pumped out by a primary crystallizer feed pump 202 and cooled down by a primary crystallizer feed cooler 203, and enters a primary crystallization circulator 204; slurry is pumped out of the primary crystallization circulator 204 by a crystallization circulating pump 205, and then enters an external circulation crystallization cooler 206, and heat of crystallization is removed from the slurry under a cooling effect of deionized water; the slurry flowing out of the primary crystallization circulator 204 is continuously delivered to a first-stage primary pressurized separating unit 207 for solid-liquid separation through a flow control valve; the phenol for washing a filter cake is a second-stage centrifuge filtrate from a slurry mixing washing mother solution pump 214, and the mother solution separated from the slurry and the washing solution enter a mother solution/washing solution tank 208 of the primary separating unit, and are pumped to a mother solution receiving tank 401 through a dephenolization feed pump 218; the phenol from a phenol discharge pump 311 is introduced from the primary separating unit, and and the phenol and the filtered adduct crystals undergo mixing and washing in a slurry mixing washing tank 210; the slurry undergoing slurry mixing is respectively pumped to a second-stage primary pressurized separating unit 212 for solid-liquid separation through a corresponding slurry pump 211; a filtrate enters a slurry mixing centrifuge mother solution tank 213, is pumped out by the slurry mixing washing mother solution pump 214 and is respectively delivered to the first-stage primary pressurized separating unit 207 and a secondary pressurized separating unit 423 for washing the phenol; the filter cake is removed by a centrifuge and fed into a melt crystallizer 215; a material in the melt crystallizer 215 is pumped to a melt heater 217 by a corresponding melt crystallizer circulating pump 216 to be heated, and then returns to the melt crystallizer 215. An operating pressure of the first-stage primary pressurized separating unit 207 and an operating pressure of the second-stage primary pressurized separating unit 212 are respectively 450 kPaA and 500 kPaA.

An adduct molten solution from a dephenolization feed pump 218 enters a falling film dephenolizer 301, is vaporized under the heating of water vapor, and enters a gas-liquid separation tank 302; a liquid phase enters a tower overhead of a stripper dephenolizer (305); a gaseous phase is condensed by a stripper dephenolization tower condenser 303, tail gas enters the vacuum system, and a condensate enters a phenol tank 310; fresh phenol from a tank zone enters a fresh phenol tank 313 at flow of 45810 kg/h, is pumped out by a fresh phenol pump 314, one part of the fresh phenol is fed into the phenol tank 310 after being metered, and the remaining fresh phenol is delivered to a phenol recycling tank 416; the phenol in the phenol tank 310 is pumped out by a phenol discharge pump 311, cooled down to a set value by a phenol washing cooler 312, and is then delivered to the slurry mixing washing tank 210 for washing; the water vapor enters a tower bottom of the stripper dephenolization tower 305 as stripped vapor after being heated by an electrically heated stripper vapor superheater 304; liquid bisphenol A yielded at the tower bottom is pumped out by a BPA melt pump 315 and then formed and granulated, and the granulated bisphenol A enters a packaging machine 316 for packaging, so as to yield a bisphenol A product; a gaseous phase at a tower overhead enters a tower bottom of a phenol absorption tower 306 and performs gas-liquid mass transfer with the absorbed phenol added from a tower overhead; the gas discharged from the tower overhead enters a phenol absorption tower condenser 309, tail gas enters the vacuum system, a condensate is aqueous phenol, and the aqueous phenol enters an ethylbenzene tank 526; and tower bottoms are a bisphenol A phenol solution, and are pumped to a sprayed phenol cooler 308 for cooling through a phenol circulating pump 307, and then returns to the tower overhead of the phenol absorption tower 306.

A material from a mother solution receiving tank 401 is sequentially pumped to the feed preheaters 403,404 through a falling film evaporator feed pump 402; the first-stage feed preheater 403 is a thermo-coupled heat exchanger, and a heating medium is a gaseous phase material from a third-stage gas-liquid separation tank 411; the second-stage feed preheater 404 is also a thermo-coupled heat exchanger, and a heating medium is the phenol from a phenol recycling pump 419; the material is divided into two parts by weight in proportion after being preheated at two stages, one part of the material enters a high-pressure pressurized falling film evaporator 406 and is vaporized under the heating of the water vapor, a gas-liquid two-phase material flowing out of a lower portion of the pressurized falling film evaporator 406 enters a first-stage gas-liquid separation tank 407, and an operating temperature and an operating pressure of the pressurized falling film evaporator 406 are respectively 152°C and 34 kPaA; the liquid phase material is pumped to a third-stage gas-liquid separation tank 411 through a first-stage separation tank discharge pump 410, the gaseous phase material is used as a heating medium, and enters a low-pressure falling film evaporator 405, and the condensed gaseous phase is separated from the condensed liquid phase in a second-stage gas-liquid separation tank 408; the liquid phase enters the phenol recycling tank 416, the gaseous phase enters a tail gas condenser 409 and is further condensed through the circulating water, a condensate enters the phenol recycling tank 416, and tail gas is delivered to the vacuum system; the other part of the preheated mother solution is heated and vaporized in the low-pressure falling film evaporator 405, a gas-liquid two-phase material flowing out of a lower portion of the low-pressure falling film evaporator 405 enters the third-stage gas-liquid separation tank 411, and an operating temperature and an operating pressure of the low-pressure falling film evaporator 405 are respectively 113°C and 7 kPaA; one part of the gaseous phase material, serving as a heating medium, enters the first-stage feed preheater 403, the redundant gaseous phase material is condensed in a condenser 414 under the action of the circulating water, tail gas of the first-stage feed preheater 403 and tail gas of the condenser 414 converge and then enter an aftercooler (415), the tail gas is further condensed under the action of the circulating water and returns to the vacuum system, and a condensate of the first-stage feed preheater 403, a condensate of the condenser 414 and a condensate of the aftercooler 415 automatically flow to the phenol recycling tank 416; the material in the phenol recycling tank 416 is pumped into a phenol recycling storage tank 418 through a phenol discharge pump 417, then pumped out through the phenol recycling pump 419, and delivered to an outlet of the first-stage cooler 101 of the condensation reactor and an outlet of the phenol circulating pump 307 respectively after being cooled down through heat exchange by the second-stage feed preheater 404 and the cracking feed preheater (601); the liquid phase material in the third-stage gas-liquid separation tank 411 is a concentrated bisphenol A phenol solution, is pumped out by a crystallizer feed pump 412 and cooled down by the circulating water through a crystallizer feed cooler (413), and then enters a recycling crystallizer 420; adduct slurry is pumped out of the recycling crystallizer 420 by a crystallization circulating pump 422, enters a crystallization cooler 421, and is cooled down by deionized water to remove heat of crystallization from the slurry; the slurry flowing out of the recycling crystallizer (420) is continuously delivered to a secondary pressurized separating unit 423 for solid-liquid separation; the phenol used to wash the filter cake is the second-stage centrifuge filtrate from the slurry mixing washing mother solution pump 214; the mother solution separated from the secondary pressurized separating unit 423 enters a mother solution receiving tank 429, is pumped out by a mother solution discharge pump 430, and is preheated by a mother solution heater 431, one part of the preheated mother solution is delivered to an isomerization reactor 432 for converting 2, 4-bisphenol A, triphenol, chroman and the like therein into bisphenol A, with a temperature at an inlet being 72°C, and then the bisphenol A enters the mother solution receiving tank (401); the other part of the preheated mother solution is delivered to the cracking and rearrangement unit 600# for cracking and rearrangement; a washing solution from the secondary pressurized separating unit 423 enters a washing solution receiving tank 424 and is pumped to the mother solution receiving tank (401) through a washing solution discharge pump 425; one part of the metered reaction liquid from the condensation reaction concentrated solution discharge pump (121) is introduced from the primary separating unit, and mixed with the adduct crystals that have been filtered in a melt crystallizer 426; the material in the melt crystallizer (426) is pumped to a melt crystallizer heater 428 by the corresponding melt crystallizer circulating pump 427 to be heated, and then returns to the melt crystallizer 426; and the material in the melt crystallizer 426) is pumped to the crystallization feed buffer tank 201 through the melt crystallizer circulating pump 427.

A dehydration tower 502 is an azeotropic rectification tower, and ethylbenzene is an entrainer; the material from the light component discharge pump 121 is mixed with a material from an ethylbenzene feed pump 527, and the mixture enters the dehydration tower (502) after being preheated by low-pressure vapor in a dehydration tower feed preheater 501; gas from a tower overhead directly enters an acetone recycling tower 504, tower bottoms are ethylbenzene-containing phenol, and the tower bottoms are delivered to a phenol recycling tower 519 through a tower kettle discharge pump 518; gas from a tower overhead of the acetone recycling tower 504 is condensed through an acetone tower condenser 506 and an aftercooler 507, and tail gas enters the vacuum system; a condensate enters an acetone tower reflux tank 508 and is pumped out by an acetone tower reflux pump 509, one stream is delivered to the tower overhead of the acetone recycling tower to be used as reflux, and the other stream is used as recycled acetone and is delivered to an inlet of the second-stage feed pump 110 of the reactor in a condensation reaction unit 100#; a material in a tower kettle enters a phase splitter 510 to form a water phase and an organic phase; the water phase material is pumped out by a water phase discharge pump 511 and cooled down by coolers (512) and (513), and then enters a phase splitter 514 for further phase splitting; the organic phase automatically flows to the phase splitter 510, and the water phase is used as effluent of the device to be pumped out of a boundary region by an effluent pump 516 for biochemical treatment; the organic phase of the phase splitter 510 is pumped out by an ethylbenzene discharge pump 515, one part of the organic phase returns to the tower overhead of the dehydration tower 502 as reflux, and the other part of the organic phase is cooled down by an ethylbenzene cooler 517 and then enters a vacuum pump as a supplementary working liquid of an ethylbenzene liquid ring vacuum pump; the amount of rising vapor in the tower is controlled by adjusting the flow of heated vapor of an acetone tower reboiler 505; a material from the tower kettle discharge pump 518 of the dehydration tower 502 enters the phenol recycling tower 519; gas from the tower overhead is condensed through a phenol tower condenser (522) and a phenol tower aftercooler 523, tail gas enters the vacuum system, and a condensate enters a phenol tower reflux tank 524; one part of the condensate is used as reflux and returns to the tower overhead through a reflux pump 525 of the phenol recycling tower, the remaining condensate overflows to an ethylbenzene tank 526, the ethylbenzene tank 526 also receives aqueous phenol from the dephenolization condenser 309, displaced ethylbenzene from the vacuum system and supplemented ethylbenzene, and the material in the ethylbenzene tank 526 is pumped to the dehydration tower 502 for feeding through the ethylbenzene feed pump 527; a tower kettle contains the ethylbenzene-free phenol, and the phenol is pumped to the phenol recycling storage tank 416 through a tower kettle discharge pump 521 of the phenol recycling tower; and the amount of rising vapor in the tower is controlled by adjusting the flow of the heated vapor of a phenol tower reboiler 520.

The mother solution from the mother solution discharge pump 430 enters a falling film evaporator 602 after being preheated by the cracking feed preheater 601; the cracking feed preheater 601 is a thermo-coupled heat exchanger and a heating medium is recycled phenol from the second-stage feed preheater 404; the mother solution is vaporized in the falling film evaporator 602 under the heating of water vapor, a gaseous phase material and a liquid phase material enter a gas-liquid separation tank 603, the liquid phase enters a tower kettle of a cracking reactor 610, and the gaseous phase enters a de-p-isopropyl phenol tower 604; tower bottoms of the de-p-isopropyl phenol tower 604 are a heavy component containing p-isopropyl phenol, and are discharged out of the system through a discharge pump (606) for incineration treatment; gas from a tower overhead is condensed and cooled down by a condenser (607), tail gas enters the vacuum system, a condensate enters a reflux tank 608, and is pumped out by a reflux pump 609, one part of the condensate returns to the tower overhead of the de-p-isopropyl phenol tower 604 as reflux, and the other part of the condensate enters a tower overhead of the cracking reactor 610 as reflux of the cracking reactor 610; a material overflowing from the reflux tank (608) enters a cracking product tank 614; the amount of rising vapor in the tower is controlled in the tower by adjusting the flow of the heated water vapor of a reboiler 605; after the material from the gas-liquid separation tank 603 enters the tower kettle of the cracking reactor

(610), the material is pumped to a cracking reaction heater 612 through a cracking reaction circulating pump 611 to be heated, a certain amount of alkali liquor is added as a catalyst of the cracking reaction, and the reactant material returns to the tower kettle of the cracking reactor 610; tar generated by cracking is discharged out of the system through the cracking reaction circulating pump 611 and is incinerated; gas from the tower overhead of the cracking reactor 610 is condensed and cooled through a cracking product condenser 613, tail gas enters the vacuum system, a condensate enters the cracking product tank 614, and is then pumped into a circulating cooler 616 for cooling through a rearrangement reaction feed pump 615; one part of the condensate circulates back to the cracking product tank 614, and the other part of the condensate is fed into a rearrangement reactor 617; and the rearranged material returns to the mother solution receiving tank 401.

The purity of the finally yielded bisphenol A product is more than 99.97wt%, the free phenol is less than 10 ppm, the 2,4-bisphenol A is less than 60 ppm, the triphenol is less than 25 ppm, the water is less than 480 ppm, and the consumption of standard oil is less than 196 kg per ton of the bisphenol A product.

### Embodiment 3:

Refined fresh acetone, at flow of 14000 kg/h, is respectively fed into a first-stage reactor 102 and a second-stage reactor (112) by weight in proportion; the acetone fed into the first-stage reactor 102 is mixed with recycled phenol from a cracking feed preheater 601 and a material from a first-stage circulating pump 105, and a mixture enters a first-stage cooler 101, and enters the first-stage reactors 102-104 after being cooled to 70°C by circulating water; and one part of the reaction liquid flowing out of the first-stage reactor 104 circulates back to the first-stage reactor 102 through the first-stage circulating pump 105, with circulating flow of 455000 kg/h, and the remaining reaction liquid is heated to 87°C by a dehydration flash heater 106 and enters a dehydration flash tank 107 to remove part of water from the remaining reaction liquid, with an operating pressure of the flash tank of 8 kPaA. Flash gas from the dehydration flash tank 107 is condensed and cooled down by a first flash condenser 108 and a second flash condenser (109), and a condensate containing water, acetone and phenol automatically flows to a light component receiving tank 124, while tail gas is fed into a vacuum system; a material dehydrated from the dehydration flash tank 107 is mixed with the recycled acetone and a second stream of fresh acetone, and the mixed material is then fed into a second-stage cooler 111 through a second-stage feed pump 110, and enters second-stage reactors 112-114 after being cooled to 75°C by the circulating water; a condensation reaction liquid from the second-stage reactor 114 is mixed with tower bottoms from a phenol absorption tower 306, then a mixture enters an adsorption column 115, and passes through a de-light component tower feed preheater 116 to be heated by heat exchange with tower bottoms of a de-light component tower 117 before entering the de-light component tower 117; the water, the unreacted acetone and one part of the phenol in the reaction liquid are distilled out of a tower overhead, and after condensation through a de-light component tower condenser 122 and a de-light component tower aftercooler 123, tail gas enters the vacuum system, and a condensate automatically flows into the light component receiving tank 124 and is delivered to a solvent recycling unit 500# through a discharge pump 125 for recycling the phenol and the acetone; a de-light component tower reboiler 118 is configured to concentrate a material in a tower kettle through heating by low-pressure vapor, and the concentrated material is pumped out by a tower kettle discharge pump 119 of the de-light component tower, cooled down by heat exchange in the de-light component tower feed preheater 116, and delivered to a condensation reaction liquid receiving tank 120.

A reaction concentrated solution from a condensation reaction liquid discharge pump 121 and a recycled adduct crystal molten solution of a mother solution recycling unit 400# from a melt crystallizer circulating pump 427 are mixed in a crystallization feed buffer tank 201, then pumped out by a crystallizer feed pump 202 and cooled down by a primary crystallizer feed cooler 203, and enters a primary crystallization circulator 204; slurry is pumped out of the primary crystallization circulator 204 by a crystallization circulating pump 205, and then enters a crystallization cooler 206, and heat of crystallization is removed from the slurry under a cooling effect of deionized water; the slurry flowing out of the primary crystallization circulator 204 is continuously delivered to a first-stage primary pressurized separating unit 207 for solid-liquid separation through a flow control valve; the phenol for washing a filter cake is a second-stage centrifuge filtrate from a slurry mixing washing mother solution pump 214, and the mother solution separated from the slurry and the washing solution enter a mother solution/washing solution tank (208) of the primary separating unit, and are pumped to a mother solution receiving tank (401) through a dephenolization feed pump 218; the phenol from a phenol discharge pump (311) is introduced from the primary separating unit, and and the phenol and the filtered adduct crystals undergo mixing and washing in a slurry mixing washing tank 210; the slurry undergoing slurry mixing is respectively pumped to a second-stage primary pressurized separating unit 212 for solid-liquid separation through a corresponding slurry pump 211; a filtrate enters a slurry mixing centrifuge mother solution tank 213, is pumped out by the slurry mixing washing mother solution pump 214 and is respectively delivered to the primary pressurized separating unit 207 and a secondary pressurized separating unit 423 for washing the phenol; the filter cake is removed by a centrifuge and fed into a melt crystallizer 215; a material in the melt crystallizer 215 is pumped to a melt heater 217 by a corresponding melt crystallizer circulating pump 216 to be heated, and then returns to the melt crystallizer 215. An operating pressure of the first-stage primary pressurized separating unit 207 and an operating pressure of the second-stage primary pressurized separating unit 212 are respectively 550 kPaA and 600 kPaA.

An adduct molten solution from a dephenolization feed pump 218 enters a falling film dephenolizer 301, is vaporized under the heating of water vapor, and enters a gas-liquid separation tank 302; a liquid phase enters a tower overhead of a stripper dephenolizer 305; a gaseous phase is condensed by a stripper dephenolization tower condenser 303, tail gas enters the vacuum system, and a condensate enters a phenol tank 310; fresh phenol from a tank zone enters a fresh phenol tank 313 at flow of 42756 kg/h, and is pumped out by a fresh phenol pump 314, one part of the fresh phenol is fed into the phenol tank 310 after being metered, and the remaining fresh phenol is delivered to a phenol recycling tank 416; the phenol in the phenol tank 310 is pumped out by a phenol discharge pump 311, cooled down to a set value by a phenol washing cooler 312, and is then delivered to the slurry mixing washing tank 210 for washing; the water vapor enters a tower bottom of the stripper dephenolization tower 305 as stripped vapor after being heated by an electrically heated stripper vapor superheater 304; liquid bisphenol A yielded at the tower bottom is pumped out by a BPA melt pump 315 and then formed and granulated, and the granulated bisphenol A enters a packaging machine 316 for packaging, so as to yield a bisphenol A product; a gaseous phase at a tower overhead enters a tower bottom of a phenol absorption tower 306 and performs gas-liquid mass transfer with the absorbed phenol added from a tower overhead; the gas discharged from the tower overhead enters a phenol absorption tower condenser 309, tail gas enters the vacuum system, a condensate is aqueous phenol, and the aqueous phenol enters an ethylbenzene tank 526; and tower bottoms are a bisphenol A phenol solution, and are pumped to a sprayed phenol cooler 308 for cooling through a phenol circulating pump 307, and then returns to the tower overhead of the phenol absorption tower 306.

A material from a mother solution receiving tank 401 is sequentially pumped to the feed preheaters 403,404 through a falling film evaporator feed pump 402; the first-stage feed preheater 403 is a thermo-coupled heat exchanger, and a heating medium is a gaseous phase material from a third-stage gas-liquid separation tank 411; the second-stage feed preheater 404 is also a thermo-coupled heat exchanger, and a heating medium is the phenol from a phenol recycling pump 419; the material is divided into two parts by weight in proportion after being preheated at two stages, one part of the material enters a high-pressure pressurized falling film evaporator 406 and is vaporized under the heating of the water vapor, a gas-liquid two-phase material flowing out of a lower portion of the pressurized falling film evaporator 406 enters a first-stage gas-liquid separation tank 407, and an operating temperature and an operating pressure of the pressurized falling film evaporator 406 are respectively 154°C and 35 kPaA; the liquid phase material is pumped to a gas-liquid separation tank 411 through a first-stage separation tank discharge pump 410, the gaseous phase material is used as a heating medium, and enters a low-pressure falling film evaporator 405, and the condensed gaseous phase is separated from the condensed liquid phase in a second-stage gas-liquid separation tank 408; the liquid phase enters the phenol recycling tank 416, the gaseous phase enters a tail gas condenser 409 and is further condensed through the circulating water, a condensate enters the phenol recycling tank 416, and tail gas is delivered to the vacuum system; the other part of the preheated mother solution is heated and vaporized in the low-pressure falling film evaporator 405, a gas-liquid two-phase material flowing out of a lower portion of the low-pressure falling film evaporator 405 enters the third-stage gas-liquid separation tank 411, and an operating temperature and an operating pressure of the low-pressure falling film evaporator 405 are respectively 114°C and 8 kPaA; one part of the gaseous phase material, serving as a heating medium, enters the feed preheater 403, the redundant gaseous phase material is condensed in a condenser 414 under the action of the circulating water, tail gas of the feed preheater 403 and tail gas of the condenser 414 converge and then enter an aftercooler 415, the tail gas is further condensed under the action of the circulating water and returns to the vacuum system, and a condensate of the first-stage feed preheater 403, a condensate of the condenser 414 and a condensate of the aftercooler 415 automatically flow to the phenol recycling tank 416; the material in the phenol recycling tank 416 is pumped into a phenol recycling storage tank 418 through a phenol discharge pump 417, then pumped out through the phenol recycling pump 419, and delivered to an outlet of the first-stage cooler 101 of the condensation reactor and an outlet of the phenol circulating pump 307 respectively after being cooled down through heat exchange by the second-stage feed preheater 404 and the cracking feed preheater 601; the liquid phase material in the third-stage gas-liquid separation tank 411 is a concentrated bisphenol A phenol solution, is pumped out by a crystallizer feed pump 412 and cooled down by the circulating water through a crystallizer feed cooler 413, and then enters a recycling crystallizer 420; adduct slurry is pumped out of the recycling crystallizer 420 by a crystallization circulating pump 422, enters a crystallization cooler 421, and is cooled down by deionized water to remove heat of crystallization from the slurry; the slurry flowing out of the recycling crystallizer 420 is continuously delivered to a secondary pressurized separating unit 423 for solid-liquid separation; the phenol used to wash the filter cake is the second-stage centrifuge filtrate from the slurry mixing washing mother solution pump 214; the mother solution separated from the secondary pressurized separating unit 423 enters a mother solution receiving tank 429, is pumped out by a mother solution discharge pump 430, and is preheated by a mother solution heater 431, one part of the preheated mother solution is delivered to an isomerization reactor 432 for converting 2, 4-bisphenol A, triphenol, chroman and the like therein into bisphenol A, with a temperature at an inlet being 75°C, and then the bisphenol A enters the mother solution receiving tank 401; the other part of the preheated mother solution is delivered to the cracking and rearrangement unit 600#; a washing solution from the secondary pressurized separating unit 423 enters a washing solution receiving tank 424 and is pumped to the mother solution receiving tank 401 through a washing solution discharge pump 425; one part of the metered reaction liquid from the condensation reaction concentrated solution discharge pump 121 is introduced from the primary separating unit, and mixed with the adduct crystals that have been filtered in a melt crystallizer 409; the material in the melt crystallizer 409 is pumped to a heater 428 by the corresponding melt crystallizer circulating pump 427 to be heated, and then returns to the melt crystallizer 409; and the material in the melt crystallizer 409 is pumped to the crystallization feed buffer tank 201 through the melt crystallizer circulating pump 427.

A dehydration tower 502 is an azeotropic rectification tower, and ethylbenzene is an entrainer; the material from the light component discharge pump 121 is mixed with a material from an ethylbenzene feed pump 527, and the mixture enters the dehydration tower 502 after being preheated by low-pressure vapor in a dehydration tower feed preheater 501; gas from a tower overhead directly enters an acetone recycling tower 504, tower bottoms are ethylbenzene-containing phenol, and the tower bottoms are delivered to a phenol recycling tower 519 through a tower kettle discharge pump 518; gas from a tower overhead of the acetone recycling tower 504 is condensed through an acetone tower condenser 506 and an aftercooler 507, and tail gas enters the vacuum system; a condensate enters an acetone tower reflux tank 508 and is pumped out by an acetone tower reflux pump 509, one stream is delivered to the tower overhead of the acetone recycling tower to be used as reflux, and the other stream is used as recycled acetone and is delivered to an inlet of the second-stage feed pump 110 of the reactor in a condensation reaction unit 100#; a material in a tower kettle enters a phase splitter (510) to form a water phase and an organic phase; the water phase material is pumped out by a water phase discharge pump 511 and cooled down by coolers 512 and 513, and then enters a phase splitter 514 for further phase splitting; the organic phase automatically flows to the phase splitter 510, and the water phase is used as effluent of the device to be pumped out of a boundary region by an effluent pump 516 for biochemical treatment; the organic phase of the phase splitter 510 is pumped out by an ethylbenzene discharge pump 515, one part of the organic phase returns to the tower overhead of the dehydration tower 502 as reflux, and the other part of the organic phase is cooled down by an ethylbenzene cooler 517 and then enters a vacuum pump as a supplementary working liquid of an ethylbenzene liquid ring vacuum pump; the amount of rising vapor in the tower is controlled by adjusting the flow of heated vapor of an acetone tower reboiler 505; a material from the tower kettle discharge pump 518 of the dehydration tower 502 enters the phenol recycling tower 519; gas from the tower overhead is condensed through a phenol tower condenser 522 and a phenol tower aftercooler 523, tail gas enters the vacuum system, and a condensate enters a phenol tower reflux tank 524; one part of the condensate is used as reflux and returns to the tower overhead through a reflux pump 525 of the phenol recycling tower, the remaining condensate overflows to an ethylbenzene tank 526, the ethylbenzene tank 526 also receives aqueous phenol from the dephenolization condenser 309, displaced ethylbenzene from the vacuum system and supplemented ethylbenzene, and the material in the ethylbenzene tank 526 is pumped to the dehydration tower 502 for feeding through the ethylbenzene feed pump 527; a tower kettle contains the ethylbenzene-free phenol, and the phenol is pumped to the phenol recycling storage tank 416 through a tower kettle discharge pump 521 of the phenol recycling tower; and the amount of rising vapor in the tower is controlled by adjusting the flow of the heated vapor of a phenol tower reboiler (520.

The mother solution from the mother solution discharge pump 430 enters a falling film evaporator 602 after being preheated by the cracking feed preheater 601; the cracking feed preheater 601 is a thermo-coupled heat exchanger and a heating medium is recycled phenol from the second-stage feed preheater 404; the mother solution is vaporized in the falling film evaporator 602 under the heating of water vapor, a gaseous phase material and a liquid phase material enter a gas-liquid separation tank 603, the liquid phase enters a tower kettle of a cracking reactor 610, and the gaseous phase enters a de-p-isopropyl phenol tower 604; tower bottoms of the de-p-isopropyl phenol tower 604 are a heavy component containing p-isopropyl phenol, and are discharged out of the system through a discharge pump 606 for incineration treatment; gas from a tower overhead is condensed and cooled down by a condenser 607, tail gas enters the vacuum system, a condensate enters a reflux tank 608, and is pumped out by a reflux pump 609, one part of the condensate returns to the tower overhead of the de-p-isopropyl phenol tower 604 as reflux, and the other part of the condensate enters a tower overhead of the cracking reactor 610 as reflux of the cracking reactor 610; a material overflowing from the reflux tank 608 enters a cracking product tank 614; the amount of rising vapor in the tower is controlled in the tower by adjusting the flow of the heated water vapor of a reboiler 605; after the material from the gas-liquid separation tank 603 enters the tower kettle of the cracking reactor 610, the material is pumped to a cracking reaction heater 612 through a cracking reaction circulating pump 611 to be heated, a certain amount of alkali liquor is added as a catalyst of the cracking reaction, and the reactant material returns to the tower kettle of the cracking reactor 610; tar generated by cracking is discharged out of the system through the cracking reaction circulating pump 611 and is incinerated; gas from the tower overhead of the cracking reactor 610 is condensed and cooled through a cracking product condenser 613, tail gas enters the vacuum system, a condensate enters the cracking product tank 614, and is then pumped into a circulating cooler 616 for cooling through a rearrangement reaction feed pump 615; one part of the condensate circulates back to the cracking product tank 614, and the other part of the condensate is fed into a rearrangement reactor 617; and the rearranged material returns to the mother solution receiving tank 401.

The purity of the finally yielded bisphenol A product is more than 99.97wt%, the free phenol is less than 10 ppm, the 2,4-bisphenol A is less than 60 ppm, the triphenol is less than 25 ppm, the water is less than 480 ppm, and the consumption of standard oil is less than 195 kg per ton of the bisphenol A product.

The technical solutions disclosed and provided by the present invention can be achieved by properly changing conditions, routes and other links by those skilled in the art by referring to the content herein. Although the method and preparation technology of the present invention have been described through the preferred embodiments, related technicians obviously can change or recombine the method and technical route herein without departing from the content, spirit and scope of the present invention, so as to achieve the final preparation technology. It should be particularly noted that all the similar replacements and changes are apparent to those skilled in the art, and they are all deemed to be included in the spirit, scope and content of the present invention.

## Claims

1. A production process for synthesizing bisphenol A by a resin method, including: feeding raw material acetone, recycled acetone and circulating phenol into the condensation reaction unit (100#) for a reaction; refined fresh acetone being respectively fed into the first-stage reactor (102) and the second-stage reactor (112) by weight in proportion; mixing the acetone fed into the first-stage reactor (102) with the recycled phenol from a cracking feed preheater (601) and a material from a first-stage circulating pump (105), a mixture entering a first-stage cooler (101), and entering the first-stage reactors (102-104) after being cooled down by circulating water; one part of the reaction liquid flowing out of the first-stage reactor (104) circulating back to the first-stage reactor (102) through the first-stage circulating pump (105), and the remaining reaction liquid being heated by a dehydration flash preheater (106) and entering a dehydration flash tank (107) to remove part of water from the remaining reaction liquid; simple separating a product from an unreacted raw material mixture, feeding a reaction liquid into the first-stage adduct crystallization unit (200#), and feeding aqueous phenol into the solvent recycling unit (500#); introducing part of raw material phenol from the first-stage adduct crystallization unit for washing an upstream reaction liquid, recycled phenol from a dephenolization refining unit (300#) and the adduct crystal molten solution from a mother solution recycling unit (400#), after washing, feeding a first-stage centrifugal mother solution/washing solution and a second-stage centrifugal mother solution into the mother solution recycling unit, and feeding the adduct crystal molten solution into the dephenolization refining unit; feeding the aqueous phenol into the solvent recycling unit while yielding a final product BPA through the dephenolization refining unit; introducing part of raw material phenol from the mother solution recycling unit for washing the mother solution, the washing solution, the recycled phenol and the recycled BPA from the cracking and rearrangement unit (600#), followed by feeding the second-stage centrifugal mother solution into the cracking and rearrangement unit; adding ethylbenzene to the solvent recycling unit to serve as an entrainer, and discharging effluent out of the system while feeding the recycled acetone into the condensation reaction unit and feeding the recycled phenol into the mother solution recycling unit; and receiving the second-stage centrifugal mother solution by the cracking and rearrangement unit to perform cracking and rearrangement, feeding the recycled BPA back to the mother solution recycling unit, and delivering p-isopropyl phenol and effluent out of the system.

2. The production process for synthesizing bisphenol A by a resin method according to Claim 1, **characterized in that** the condensation reaction unit (100#) comprises:
flash gas from the dehydration flash tank (107) is condensed and cooled down by a first flash condenser (108) and a second flash condenser (109), and a condensate containing water, acetone and phenol automatically flows to a light component receiving tank (124), while tail gas is fed into a vacuum system; a material dehydrated from the dehydration flash tank (107) is mixed with the recycled acetone and a second stream of fresh acetone, the mixed material is then fed into a second-stage cooler (111) through a second-stage feed pump (110), and enters second-stage reactors (112-114) after being cooled down by the circulating water; a condensation reaction liquid from the second-stage reactor (114) is mixed with tower bottoms from a phenol absorption tower (306), then a mixture enters an adsorption column (115), and passes through a de-light component tower feed preheater (116) to be heated by tower bottoms of a de-light component tower (117), and then enters the de-light component tower (117); the water, the unreacted acetone and part of the phenol in the reaction liquid are distilled out of a tower overhead, and after condensation through a de-light component tower condenser (122) and a de-light component tower aftercooler (123), tail gas enters the vacuum system, a condensate automatically flows into the light component receiving tank (124) and is delivered to the solvent recycling unit (500#) through a discharge pump (125) for recycling the phenol and the acetone; and a de-light component tower reboiler (118) is configured to concentrate a material in a tower kettle through heating by low-pressure vapor, and the concentrated material is pumped out by a tower kettle discharge pump (119) of the de-light component tower, cooled down by heat exchange in the de-light component tower feed preheater (116), and delivered to a condensation reaction liquid receiving tank (120).

3. The production process for synthesizing bisphenol A by a resin method according to Claim 1, **characterized in that** the first-stage adduct crystallization unit (200#) comprises:
a reaction concentrated solution from a condensation reaction liquid discharge pump (121) and a recycled adduct crystal molten solution of the mother solution recycling unit (400#) from a melt crystallizer circulating pump (427) are mixed in a crystallization feed buffer tank (201), then pumped out by a crystallizer feed pump (202) and cooled down by a primary crystallizer feed cooler (203), and enters a primary crystallization circulator (204); slurry is pumped out of the primary crystallization circulator (204) by a crystallization circulating pump (205), and then enters a crystallization cooler (206), and heat of crystallization is removed from the slurry under a cooling effect of deionized water; the slurry flowing out of the primary crystallization circulator (204) is continuously delivered to a first-stage primary pressurized separating unit (207) for solid-liquid separation through a flow control valve; the phenol for washing a filter cake is a second-stage centrifuge filtrate from a slurry mixing washing mother solution pump (214), and the mother solution separated from the slurry and the washing solution enter a mother solution/washing solution tank (208) of the primary separating unit and pumped to a mother solution receiving tank (401) through a dephenolization feed pump (218); the phenol from a phenol discharge pump (311) is introduced from the primary separating unit, and the phenol and the filtered adduct crystals undergo mixing and washing in a slurry mixing washing tank (210); the slurry undergoing slurry mixing is respectively pumped to a second-stage primary pressurized separating unit (212) for solid-liquid separation through a corresponding slurry pump (211); a filtrate enters a slurry mixing centrifuge mother solution tank (213), is pumped out by the slurry mixing washing mother solution pump (214) and is respectively delivered to the first-stage primary pressurized separating unit (207) and a secondary pressurized separating unit (423) for washing the phenol; the filter cake is removed by a centrifuge and fed into a melt crystallizer (215); and a material in the melt crystallizer (215) is pumped to a melt heater (217) by a corresponding melt crystallizer circulating pump (216) to be heated, and then returns to the melt crystallizer (215).

4. The production process for synthesizing bisphenol A by a resin method according to Claim 1, **characterized in that** the dephenolization refining unit (300#) comprises:
an adduct molten solution from a dephenolization feed pump (218) enters a falling film dephenolizer (301), is vaporized under the heating of water vapor, and enters a gas-liquid separation tank (302); a liquid phase enters a tower overhead of a stripper dephenolization tower (305); a gaseous phase is condensed by a stripper dephenolization tower condenser (303), tail gas enters the vacuum system, and a condensate enters a phenol tank (310); fresh phenol from a tank zone enters a fresh phenol tank (313), and is pumped out by a fresh phenol pump (314), one part of the fresh phenol is fed into the phenol tank (310) after being metered, and the remaining fresh phenol is delivered to a phenol recycling tank (416); the phenol in the phenol tank (310) is pumped out by a phenol discharge pump (311), cooled down to a set value by a phenol washing cooler (312), and is then delivered to the slurry mixing washing tank (210) used for washing; the water vapor passes through an electrically heated vapor superheater (304) for heating, and then enters a tower bottom of the stripper dephenolization tower (305) as stripped vapor; liquid bisphenol A yielded at the tower bottom is pumped out by a BPA melt pump (315) and then formed and granulated, and the granulated bisphenol A enters a packaging machine (316) for packaging, so as to yield a bisphenol A product; a gaseous phase at a tower overhead enters a tower bottom of a phenol absorption tower (306) and performs gas-liquid mass transfer with the absorbed phenol added from a tower overhead; the gas discharged from the tower overhead enters a phenol absorption tower condenser (309), tail gas enters the vacuum system, a condensate is aqueous phenol, and the aqueous phenol enters an ethylbenzene tank (526); and tower bottoms are a bisphenol A phenol solution, and are pumped to a sprayed phenol cooler (308) for cooling through a phenol circulating pump (307), and then returns to the tower overhead of the phenol absorption tower (306).

5. The production process for synthesizing bisphenol A by a resin method according to Claim 1, **characterized in that** the mother solution recycling unit (400#) comprises:
a material from a mother solution receiving tank (401) is sequentially pumped to first-stage feed preheaters (403) and second-stage feed preheater (404) through a falling film evaporator feed pump (402); the first-stage feed preheater (403) is a thermo-coupled heat exchanger, and a heating medium is a gaseous phase material from a third-stage gas-liquid separation tank (411); the second-stage feed preheater (404) is also a thermo-coupled heat exchanger, and a heating medium is the phenol from a phenol recycling pump (419); the material is divided into two parts by weight after being two-stage preheated, one part of the material enters a high-pressure falling film evaporator (406) and is vaporized under the heating of the water vapor, and a gas-liquid two-phase material flowing out of a lower portion of the high-pressure falling film evaporator (406) enters a first-stage gas-liquid separation tank (407); the liquid phase material is pumped to a third-stage gas-liquid separation tank (411) through a first-stage separation tank discharge pump (410), the gaseous phase material is used as a heating medium, and enters a low-pressure falling film evaporator (405), and the condensed gaseous phase is separated from the condensed liquid phase in a second-stage gas-liquid separation tank (408); the liquid phase enters the phenol recycling tank (416), the gaseous phase enters a tail gas condenser (409) and is further condensed through the circulating water, a condensate enters the phenol recycling tank (416), and tail gas is delivered to the vacuum system; the other part of the preheated mother solution is heated and vaporized in the low-pressure falling film evaporator (405), and a gaseous phase material and a liquid phase material flowing out of a lower portion of the low-pressure falling film evaporator (405) enters the third-stage gas-liquid separation tank (411); one part of the gaseous phase material, serving as a heating medium, enters the first-stage feed preheater (403), the redundant gaseous phase material is condensed in a condenser (414) under the action of the circulating water, tail gas of the first-stage feed preheater (403) and tail gas of the condenser (414) converge and then enter an aftercooler (415), the tail gas is further condensed under the action of the circulating water and returns to the vacuum system, and a condensate of the first-stage feed preheater (403), a condensate of the condenser (414) and a condensate of the aftercooler (415) automatically flow to the phenol recycling tank (416); the material in the phenol recycling tank (416) is pumped into a phenol recycling storage tank (418) through a phenol discharge pump (417), then pumped out through the phenol recycling pump (419), and delivered to an outlet of the first-stage cooler (101) of the condensation reactor and an outlet of the phenol circulating pump (307) respectively after being cooled down through heat exchange by the second-stage feed preheater (404) and the cracking feed preheater (601); the liquid phase material in the third-stage gas-liquid separation tank (411) is a concentrated bisphenol A phenol solution, is pumped out by a crystallizer feed pump (412) and cooled down by the circulating water through a crystallizer feed cooler (413), and then enters a recycling crystallizer (420); adduct slurry is pumped out of the recycling crystallizer (420) by a crystallization circulating pump (422) and then enters a crystallization cooler (421), and is cooled down by deionized water to remove heat of crystallization from the slurry; the slurry flowing out of the recycling crystallizer (420) is continuously delivered to a secondary pressurized separating unit (423) for solid-liquid separation; the phenol used to wash the filter cake is the second-stage centrifuge filtrate from the slurry mixing washing mother solution pump (214); the mother solution separated from the secondary pressurized separating unit (423) enters a mother solution receiving tank (429) and is pumped out by a mother solution discharge pump (430), and is preheated by a mother solution heater (431), one part of the preheated mother solution is delivered to an isomerization reactor (432) for converting 2,4-bisphenol A, triphenol, chroman and the like therein into bisphenol A, and then the bisphenol A enters the mother solution receiving tank (401); the other part of the preheated mother solution is delivered to the cracking and rearrangement unit 600# for cracking and rearrangement; a washing solution from the secondary pressurized separating unit (423) enters a washing solution receiving tank (424) and is pumped to the mother solution receiving tank (401) through a washing solution discharge pump (425); one part of the metered reaction liquid from the condensation reaction concentrated solution discharge pump (121) is introduced from the primary separating unit, and mixed with the adduct crystals that have been filtered in a melt crystallizer (409); the material in the melt crystallizer (409) is pumped to a heater (428) by a corresponding melt crystallizer circulating pump (427) to be heated, and then returns to the melt crystallizer (409); and the material in the melt crystallizer (409) is pumped to the crystallization feed buffer tank (201) through the melt crystallizer circulating pump (427).

6. The production process for synthesizing bisphenol A by a resin method according to Claim 1, **characterized in that** the solvent recycling unit (500#) comprises:
a dehydration tower (502) is an azeotropic rectification tower, and ethylbenzene is an entrainer; the material from the light component discharge pump (125) is mixed with a material from an ethylbenzene feed pump (527), and the mixture enters the dehydration tower (502) after being preheated by low-pressure vapor in a dehydration tower feed preheater (501); gas from a tower overhead directly enters an acetone recycling tower (504), tower bottoms are ethylbenzene-containing phenol, and the tower bottoms are delivered to a phenol recycling tower (519) through a tower kettle discharge pump (518); gas from a tower overhead of the acetone recycling tower (504) is condensed through an acetone tower condenser (506) and an aftercooler (507), and tail gas enters the vacuum system; a condensate enters an acetone tower reflux tank (508) and is pumped out by an acetone tower reflux pump (509), one stream is delivered to the tower overhead of the acetone recycling tower to be used as reflux, and the other stream is used as recycled acetone and is delivered to an inlet of the second-stage feed pump (110) of the reactor in the condensation reaction unit (100#); a material in a tower kettle enters a phase splitter (510) to form a water phase and an organic phase; the water phase material is pumped out by a water phase discharge pump (511) and cooled down by coolers (512, 513), and then enters a phase splitter (514) for further phase splitting; the organic phase automatically flows to the phase splitter (510), and the water phase is used as effluent to be pumped out of a boundary region by an effluent pump (516) for biochemical treatment; the organic phase of the phase splitter (510) is pumped out by an ethylbenzene discharge pump (515), one part of the organic phase returns to the tower overhead of the dehydration tower (502) as reflux, and the other part of the organic phase is cooled down by an ethylbenzene cooler (517) and then enters a vacuum pump as a supplementary working liquid of an ethylbenzene liquid ring vacuum pump; the amount of rising vapor in the tower is controlled by adjusting the flow of heated vapor of a acetone tower reboiler (505); a material from the tower kettle discharge pump (518) of the dehydration tower (502) enters the phenol recycling tower (519); gas from the tower overhead is condensed through a phenol tower condenser (522) and a phenol tower aftercooler (523), tail gas enters the vacuum system, and a condensate enters a phenol tower reflux tank (524); one part of the condensate is used as reflux and returns to a tower overhead through a reflux pump (525) of the phenol recycling tower, the remaining condensate overflows to an ethylbenzene tank (526), the ethylbenzene tank (526) also receives aqueous phenol from the phenol absorption tower condenser (309), displaced ethylbenzene from the vacuum system and supplemented ethylbenzene, and the material in the ethylbenzene tank (526) is pumped to the dehydration tower (502) for feeding through the ethylbenzene feed pump (527); a tower kettle contains the ethylbenzene-free phenol, and the phenol is pumped to the phenol recycling storage tank (416) through a tower kettle discharge pump (521) of the phenol recycling tower; and the amount of rising vapor in the tower is controlled by adjusting the flow of the heated vapor of a phenol tower reboiler (520).

7. The production process for synthesizing bisphenol A by a resin method according to Claim 1, **characterized in that** the cracking and rearrangement unit (600#) comprises:
the mother solution from the mother solution discharge pump (430) enters a falling film evaporator (602) after being preheated by the cracking feed preheater (601); the cracking feed preheater (601) is a thermo-coupled heat exchanger, and a heating medium is recycled phenol from the second-stage feed preheater (404); the mother solution is vaporized in the falling film evaporator (602) under the heating of water vapor, a gaseous phase material and a liquid phase material enter a gas-liquid separation tank (603), the liquid phase enters a tower kettle of a cracking reactor (610), and the gaseous phase enters a de-p-isopropyl phenol tower (604); tower bottoms of the de-p-isopropyl phenol tower (604) are a heavy component containing p-isopropyl phenol, and are discharged out of the system through a discharge pump (606) for incineration treatment; gas from a tower overhead is condensed and cooled down by a condenser (607), tail gas enters the vacuum system, a condensate enters a reflux tank (608), and is pumped out by a reflux pump (609), one part of the condensate returns to the tower overhead of the de-p-isopropyl phenol tower (604) as reflux, and the other part of the condensate enters a tower overhead of the cracking reactor (610) as reflux of the cracking reactor (610); a material overflowing from the reflux tank (608) enters a cracking product tank (614); the amount of rising vapor in the tower is controlled by adjusting the flow of the heated water vapor of a reboiler (605); after the material from the gas-liquid separation tank (603) enters the tower kettle of the cracking reactor (610), the material is pumped to a cracking reaction heater (612) through a cracking reaction circulating pump (611) to be heated, a certain amount of alkali liquor is added as a catalyst of the cracking reaction, and the reactant material returns to the tower kettle of the cracking reactor (610); tar generated by cracking is discharged out of the system through the cracking reaction circulating pump (611) and is incinerated; gas from the tower overhead of the cracking reactor (610) is condensed and cooled through a cracking product condenser (613), tail gas enters the vacuum system, a condensate enters the cracking product tank (614), and is then pumped into a circulating cooler (616) for cooling through a rearrangement reaction feed pump (615); one part of the condensate circulates back to the cracking product tank (614), and the other part of the condensate is fed into a rearrangement reactor (617); and the rearranged material returns to the mother solution receiving tank (401).
